# EUROPEAN PATENT APPLICATION

(11) **EP 2 048 244 A1**
(43) Date of publication of application: **15.04.2009**
(21) Application number: 07301441.7
(22) Date of filing: 08.10.2007
(51) Int. Cl.: C12Q 1/68

(54) **PTPL1 as a biomarker of survival in breast cancer**

(71) Applicant: INSTITUT NATIONAL DE LA SANTE ET DE LA RECHERCHE MEDICALE (INSERM), 75013 Paris (FR)
(72) Inventor: The designation of the inventor has not yet been filed
(74) Representative: Warcoin, Jacques

(57) **Abstract**

The present invention relates to the use of PTPL1 as a biomarker of survival in breast cancer. More particularly, the present invention relates to a method for predicting the survival of a breast cancer patient comprising determining the expression level of the PTPL1 gene in a biological sample obtained from said patient.

## Description

### FIELD OF THE INVENTION

The present invention relates to the use of PTPL1 as a biomarker of survival in breast cancer.

### BACKGROUND OF THE INVENTION

Breast cancer affects about one million times per year, and represents the most common form of cancer in females, affecting approximately 10% of all women at some stage of their life in the Western world. Men can also develop breast cancer, although their risk is less than 1 in 1000. Breast cancer risk is modified by many different factors. Besides gender, the main risk factors of getting breast cancer are increasing age, having a family history of breast cancer and exposure to endogenous or exogenous estrogens. Actually, factors contributing to an increased cumulative estrogen exposure have clearly been shown to increase the risk of breast cancer development.
Despite efforts to improve treatment and management of cancer patients, survival in cancer patients has not improved over the past two decades for many cancer types. Optimal therapy will be based on a combination of diagnostic and prognostic information. An accurate and reproducible diagnostic test is needed to provide a prognostic assessment that will provide specific information regarding survival.
In breast cancer, the clinical and biological variables commonly used to predict the survival of primary chirurgical treatments are regional lymph nodes invasion, histological grade, and hormone receptor expression. All these parameters are well recognized prognostic and predictive factors. Additionally, the expression of new markers, associated to proliferation (Ki-67) and cell cycle (cyclin E, cyclin D1) (Esteva FJ. et al. 2004), to mitogenic and survival pathways (tyrosine kinase receptors HERs) (Pawlowski V. et al. 2000) and to invasion process (uPA, Cathepsin D) (Peyrat JP. et al. 1998; Thorpe SM et al 19989) has also been linked to the survival of breast cancer patients or to the response to hormonal or cytotoxic therapies. However there is an existing need to provide further biomarkers that will strengthen the prediction of survival in breast cancer patients.

### SUMMARY OF THE INVENTION

The present invention relates to a method for predicting the survival of a breast cancer patient comprising determining the expression level of the PTPL1 gene in a biological sample obtained from said patient.

### DETAILED DESCRIPTION OF THE INVENTION

### Definitions:

The term "PTPL1" has its general meaning in the art and refers to the protein tyrosine phosphatase PTPL1. PTPL1 is also known as hPTP1 E, PTP-BAS, FAP-1, or PTPN13. PTPL1 can be from any source, but typically is a mammalian (e.g., human and non-human primate) PTPL1, particularly a human PTPL1. The "term PTPL1 gene" refers to any nucleotide sequence encoding the PTPL1 mRNA and protein, such as a genomic DNA sequence and any naturally occurring PTPL1 and variants and modified forms thereof. It can also encompass artificial sequences such as cDNA encoding the PTPL1 mRNA and protein. Exemplary human native PTPL1 nucleotide sequences are provided in GenBank database under accession numbers NC_000004 for PTPL1 gene, NM_080683 (SEQ ID NO:1), NM_080684 (SEQ ID NO:2), NM_080685 (SEQ ID NO:3) and NM_006264 (SEQ ID NO:4) for PTL1 mRNA (cDNA). The term "PTPL1 mRNA" has its general meaning in the art and refers to the messenger RNA which is synthesized upon expression of the PTPL1 gene. The term "PTPL1 protein" refers to the amino acid sequence resulting from the expression of the PTPL1 gene, and any naturally occurring PTPL1 and variants and modified forms thereof. Exemplary human native PTPL1 amino acid sequences are provided in GenPept database under accession numbers, NP_542414 (SEQ ID NO:5), NP_542415 (SEQ ID NO:6), NP_542416 (SEQ ID NO:7) and NP_006255 (SEQ ID NO:8).

The term "expression" when used in the context of expression of a gene or nucleic acid refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of a mRNA. Gene products also include messenger RNAs which are modified, by processes such as capping, polyadenylation, methylation, and editing, and proteins modified by, for example, methylation, acetylation, phosphorylation, ubiquitination, sumoylation, ADP-ribosylation, myristilation, and glycosylation.
The term "anti-PTPL1 antibody" refers to an antibody or a fragment thereof which recognizes PTPL1.
As used herein, the term "predetermined value" refers to the amount of PTPL1 in biological samples obtained from the general population or from a selected population of subjects. For example, the predetermined value may be of the amount of PTPL1 obtained from breast cancer patients who have survived. The predetermined value can be a threshold value, or a range. The predetermined value can be established based upon comparative measurements between breast cancer patients who have survived and breast cancer patients who have not survived.
The term "patient" as used herein denotes a mammal such as a rodent, a feline, a canine and a primate. Preferably, a patient according to the invention is a human. The term "breast cancer patient" denotes a patient who is affected with a breast cancer.
The term "biological sample" means any biological sample derived from a patient. As an example, said biological sample refers to a breast biopsy which has been removed from the body of a patient prior to executing the method of the invention, or to a cell or cell line obtained from such a breast biopsy.
The term "biomarker", as used herein, refers generally to a molecule, i.e., a gene (or nucleic acid encoding said gene), protein, the expression of which in a biological sample from a patient can be detected by standard methods in the art (as well as those disclosed herein), and is predictive or denotes a condition of the subject from which it was obtained.

### Predictive methods of the invention:

The present invention relates to a method for predicting the survival of a breast cancer patient comprising determining the expression level of the PTPL1 gene in a biological sample obtained from said patient.

The biological sample may contain nucleic acids or proteins. Examples of such biological samples include tissues, cell samples, organs, biopsies, etc. The biological sample may result from a biopsy, and more specifically from a breast biopsy. Breast cancer biopsy is a well known technique in the art, and may involve the use of a needle.
Total nucleic acids or proteins can be easily extracted from cell or tissue samples. The biological sample may be treated prior to its use, e.g. in order to render nucleic acids or proteins available. Techniques of cell or protein lysis, concentration or dilution of nucleic acids, are known by the skilled person.

In one embodiment, the invention relates to a method for predicting the survival of breast cancer patient comprising determining the quantity of mRNA encoding PTPL1 in a cell or tissue sample obtained from said patient.
Determination of the expression level of a gene can be performed by a variety of techniques. Generally, the expression level as determined is a relative expression level.
More preferably, the determination comprises contacting the sample with selective reagents such as probes, primers or ligands, and thereby detecting the presence, or measuring the amount of nucleic acids of interest originally in the sample.
In a preferred embodiment, the expression level may be determined by measuring the quantity of mRNA in a biological sample of said patient.
Advantageously, the method of the invention further comprise a step of comparing the concentration of the PTPL1 mRNA with a predetermined value. Said comparison is indicative of the survival for the breast cancer patient, with an overexpression of the PTPL1 mRNA in the biological sample compared to the predetermined value being associated with a longer survival prognostic.
Methods for determining the quantity of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA is then detected by hybridization (e. g., Northern blot analysis) and/or amplification (e.g., RT-PCR). In a preferred embodiment, the expression level of the PTPL1 gene is determined by RT-PCR, preferably quantitative or semi-quantitative RT-PCR, even more preferably real-time quantitative or semi-quantitative RT-PCR.
Other methods of amplification include ligase chain reaction (LCR), transcription-mediated amplification (TMA), strand displacement amplification (SDA) and nucleic acid sequence based amplification (NASBA).
Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably 85% identical and even more preferably 90-95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).
Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).
The nucleic acid primers or probes used in the above amplification and detection method may be assembled as a kit. Such a kit includes consensus primers and molecular probes. A preferred kit also includes the components necessary to determine if amplification has occurred. The kit may also include, for example, PCR buffers and enzymes; positive control sequences, reaction control primers; and instructions for amplifying and detecting the specific sequences.

In another embodiment, the invention relates to a method for predicting the survival of a breast cancer patient comprising measuring the quantity of the PTPL1 protein in a biological sample obtained from said patient.
In a particular embodiment, the methods of the invention comprise contacting the biological sample with a binding partner capable of selectively interacting with the PTPL1 protein present in the biological sample.
The binding partner may be an antibody that may be polyclonal or monoclonal, preferably monoclonal. In another embodiment, the binding partner may be an aptamer.
Polyclonal antibodies of the invention or a fragment thereof can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred.
Monoclonal antibodies of the invention or a fragment thereof can be prepared and isolated using any technique that provides for the production of antibody molecules by continuous cell lines in culture. Techniques for production and isolation include but are not limited to the hybridoma technique originally described by Kohler and Milstein (1975); the human B-cell hybridoma technique (Cote et al., 1983); and the EBV-hybridoma technique (Cole et al. 1985).
Alternatively, techniques described for the production of single chain antibodies (see e.g. U.S. Pat. No. 4,946,778) can be adapted to produce anti-PTPL1, single chain antibodies. Antibodies useful in practicing the present invention also include anti-PTPL1 fragments including but not limited to F(ab')2 fragments, which can be generated by pepsin digestion of an intact antibody molecule, and Fab fragments, which can be generated by reducing the disulfide bridges of the F(ab')2 fragments. Alternatively, Fab and/or scFv expression libraries can be constructed to allow rapid identification of fragments having the desired specificity to PTPL1. For example, phage display of antibodies may be used. In such a method, single-chain Fv (scFv) or Fab fragments are expressed on the surface of a suitable bacteriophage, e. g., M13. Briefly, spleen cells of a suitable host, e. g., mouse, that has been immunized with a protein are removed. The coding regions of the VL and VH chains are obtained from those cells that are producing the desired antibody against the protein. These coding regions are then fused to a terminus of a phage sequence. Once the phage is inserted into a suitable carrier, e. g., bacteria, the phage displays the antibody fragment. Phage display of antibodies may also be provided by combinatorial methods known to those skilled in the art. Antibody fragments displayed by a phage may then be used as part of an immunoassay.
Examples of commercially available monoclonal antibodies for PTPL1 include those obtained from Leica Microsystems under the reference AC21.
Examples of commercially polyclonal antibodies for PTPL1 include those obtained from Santa Cruz Biotech under the references sc-15356, sc-1138, or sc-1729; from R&D systems under the reference ref AF3577; or from Abcam under the references Ab26218 and Ab 31017.
In another embodiment, the binding partner may be an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. 1997. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consist of conformationally constrained antibody variable regions displayed by a platform protein, such as E. coli Thioredoxin A, that are selected from combinatorial libraries by two hybrid methods (Colas et al., 1996).

The binding partners of the invention such as antibodies or aptamers, may be labelled with a detectable molecule or substance, such as a fluorescent molecule, a radioactive molecule or any others labels known in the art. Labels are known in the art that generally provide (either directly or indirectly) a signal.
As used herein, the term "labelled", with regard to the antibody, is intended to encompass direct labelling of the antibody or aptamer by coupling (i.e., physically linking) a detectable substance, such as a radioactive agent or a fluorophore (e.g. fluorescein isothiocyanate (FITC) or phycoerythrin (PE) or Indocyanine (Cy5)) to the antibody or aptamer, as well as indirect labelling of the probe or antibody by reactivity with a detectable substance. An antibody or aptamer of the invention may be labelled with a radioactive molecule by any method known in the art. For example radioactive molecules include but are not limited radioactive atom for scintigraphic studies such as I123, I124, In111, Re186, Re188.

The aforementioned assays may involve the binding of the binding partner (ie. Antibody or aptamer) to a solid support. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

PTPL1 protein may be measured by using standard immunodiagnostic techniques, including immunoassays such as competition, direct reaction, or sandwich type assays. Such assays include, but are not limited to, agglutination tests; enzyme-labelled and mediated immunoassays, such as ELISAs; biotin/avidin type assays; radioimmunoassays; immunoelectrophoresis; immunoprecipitation.

More particularly, an ELISA method can be used, wherein the wells of a microtiter plate are coated with a set of anti-PTPL1 antibodies. A biological sample containing or suspected of containing PTPL1 is then added to the coated wells. After a period of incubation sufficient to allow the formation of antibody-antigen complexes, the plate(s) can be washed to remove unbound moieties and a detectably labelled secondary binding molecule added. The secondary binding molecule is allowed to react with any captured sample marker protein, the plate washed and the presence of the secondary binding molecule detected using methods well known in the art.

Alternatively an immunohistochemistry (IHC) method may be used. IHC specifically provides a method of detecting targets in a sample or tissue specimen in situ. The overall cellular integrity of the sample is maintained in IHC, thus allowing detection of both the presence and location of the targets of interest. Typically a sample is fixed with formalin, embedded in paraffin and cut into sections for staining and subsequent inspection by light microscopy. Current methods of IHC use either direct labeling or secondary antibody-based or hapten-based labeling. Examples of known IHC systems include, for example, EnVision(TM) (DakoCytomation), Powervision(R) (Immunovision, Springdale, AZ), the NBA(TM) kit (Zymed Laboratories Inc., South San Francisco, CA), HistoFine(R) (Nichirei Corp, Tokyo, Japan).
In particular embodiment, a tissue section (ie a breast tissue sample) may be mounted on a slide or other support after incubation with anti-PTPL1 antibodies. Then, microscopic inspections in the sample mounted on a suitable solid support may be performed. For the production of photomicrographs, sections comprising samples may be mounted on a glass slide or other planar support, to highlight by selective staining the presence of PTPL1 protein.
Therefore IHC samples may include, for instance: (a) preparations comprising fresh tissues or cells isolated form said tissues (b) fixed and embedded said tissue or cells samples and (c) detecting PTPL1 protein in said tissues or cells samples. In some embodiments, an IHC staining procedure may comprise steps such as: cutting and trimming tissue, fixation, dehydration, paraffin infiltration, cutting in thin sections, mounting onto glass slides, baking, deparaffination, rehydration, antigen retrieval, blocking steps, applying primary anti-PTPL1 antibody, washing, applying secondary antibody (optionally coupled to a suitable detectable label), washing, counter staining, and microscopic examination.

Measuring the PTPL1 protein (with or without immunoassay-based methods) may also include separation of the compounds: centrifugation based on the compound's molecular weight; electrophoresis based on mass and charge; HPLC based on hydrophobicity; size exclusion chromatography based on size; and solid-phase affinity based on the compound's affinity for the particular solid-phase that is used. Once separated, PTPL1 may be identified based on the known "separation profile" e. g., retention time, for that compound and measured using standard techniques.

Alternatively, the separated compounds may be detected and measured by, for example, a mass spectrometer.

In one embodiment, the method of the invention further may comprise a step of comparing the concentration of the PTPL1 protein with a predetermined value. Said comparison is indicative of the survival in the breast cancer patient, with an overexpression of the PTPL1 protein in the biological sample compared to the predetermined value being associated with a longer survival prognostic.

Another object of the invention is PTPL1 as a biomarker of survival in breast cancer.

The method of the invention may be thus useful for classifying breast cancer patients and then may be used to choose the accurate treatment. For example, patients with a low score of survival may receive a more intensive treatment and attention compared to patient with a high score. Such method may thus help the physician to make a choice on a therapeutic treatment which can accordingly consist in administering accurate drugs to the patients.

Yet another object of the invention relates to a kit for predicting the survival of a breast cancer patient, comprising means for measuring the expression of PTPL1 gene in a biological sample obtained from the sample. The kit may include probes and primers as above described. The kit may include an antibody, or a set of antibodies as above described. In a particular embodiment, the antibody or set of antibodies are labelled as above described. The kit may also contain other suitably packaged reagents and materials needed for the particular detection protocol, including solid-phase matrices, if applicable, and standards.

The invention will further be illustrated in view of the figures and example.

### FIGURE LEGENDS

**Figure1****:** Kaplan-Meier Estimates of Overall Survival for all 291 Patients. The clinical cutoff value corresponds to the upper quartile.
**Figure2****:** Kaplan-Meier Estimates of Overall Survival and relapse free survival for the 155 Node positive Patients. The clinical cutoff value corresponds to the upper quartile.

### EXAMPLE

### Material and Methods :

**Patients:** The pilot study involved 59 breast tumour samples; the mean age of the patients was 59.7 years (range 35-81). The median duration follow-up of living patients was 7.5 years. The number of deaths was 21, and the number of relapses was 27.
To confirm the results in a larger sample set, 232 complementary breast tumours samples were included, constituting a testing set of 291 patients (Table 1).
All patients underwent surgery for locoregional disease in the Centre Oscar Lambret, the anticancer center of the north of France, between May 1989 and December 1991. The mean age of the patients was 57.9 years (range 26-90). Patients were treated by segmentectomy when the tumour was smaller than 3 cm wide and by total mastectomy if the tumour was larger or centrally located. Surgery was followed by radiation therapy. Node-positive premenopausal patients, ER negative, and PR negative postmenopausal patients received adjuvant treatment: six cycles of chemotherapy. The node-positive, ER positive, and PR positive postmenopausal patients received tamoxifen for two years. Node-negative patients received no adjuvant treatment. The median duration follow-up of living patients was 6.4 years. The number of deaths was 83, and the number of relapses was 108.
The biopsies were obtained with the agreement of the Institutional Review Board.
Both Estrogen Receptor (ER) and Progesterone Receptor (PR) were determined by the dextran-coated charcoal method, as described previously [23]. The Laboratoire d'Oncologie Moléculaire Humaine is affiliated with the European Organisation for Research and Treatment of Cancer Receptor Study Group, which undertook the quality control of the assays (Koenders T, et al. 1983).

**Table 1 : Patients and tumor characteristics (n=291)**

| Characteristics | | No of cases |
|---|---|---|
| Age | | |
| | <50 years | 69 |
| | ≥50 years | 222 |
| | | |
| Node involvement | | |
| | negative | 134 |
| | positive | 155 |
| | unknown | 2 |
| | | |
| | | |
| HPG | | |
| | I | 42 |
| | II | 111 |
| | III | 109 |
| | unkown | 29 |
| | | |
| Tumor type | | |
| | Ductular | 212 |
| | Lobular | 22 |
| | Others | 57 |
| | | |
| Tumor diameter | | |
| | < 2cm | 21 |
| | 2 to 5cm | 188 |
| | > 5cm | 68 |
| | unknown | 14 |
| | | |
| | | |
| ER | | |
| | < 10 fmol/mg protein | 88 |
| | ≥ 10 fmol/mg protein | 201 |
| | unknown | 2 |
| | | |
| PgR | | |
| | <10 fmol/mg protein | 88 |
| | ≥ 10 fmol/mg protein | 200 |
| | unknown | 3 |

**PTP mRNA expression:** Total RNA was isolated (RNeasy Mini Kit, Qiagen, France) from 40 mg of each tumour sample. Disruption and homogenisation of the tumour samples were performed using a Rotor-Stator Homogeniser (Ribolyzer, Hybaid). The amount of extracted RNA was quantified by measuring the absorbance at 260 nm. The quality of the RNA was checked by the ratio between the absorbance values at 260 nm and 280 nm, and then confirmed by electrophoresis of the RNA on a 1.5% agarose gel containing ethidium bromide. Total RNA (2 µg) was reverse transcribed into cDNA using 5µM of random hexamers (Roche) and Superscript II RNAse H- reverse transcriptase according to the manufacturer instructions (Invitrogen).
The real time Polymerase-Chain-Reaction (PCR) quantification of each gene was carried out on cDNA corresponding to 12.5 ng of total RNA using a Light Cycler 3 (Roche) with the LightCycler FastStart DNA MasterPLUS SYBR Green I Kit (Roche) according to the manufacturer instructions. Primer sequences and gene position, product length, time and temperature used for each gene are indicated in Table 2.

**Table 2: PCR conditions and primers**

| Gene | Primer sequence | Exon | Product size | Annealing Temp. |
|---|---|---|---|---|
| PTP gamma | Upper 5' GCA ACT CGA TGG CTT CGA CAA 3' (SEQ ID NO: 9) | 3 | 84bp | 61°C |
| | Lower  5' TCT TTC AGA AGG ATG GCG ACT GTT 3' (SEQ ID NO: 10) | 3-4 | | |
| LAR-Neu | Upper 5' TGG ACT CCC CAT CAT CCA AGA 3' (SEQ ID NO: 11) | 13 | 85bp | 66°C |
| | Lower  5' CCG CTG ATA GTG GTT TCA TAG TCC T 3' (SEQ ID NO: 12) | 14-15 | | |
| LAR | Upper 5' TAG CCG AGG CCC AGG AAA C 3' (SEQ ID NO: 13) | 13-15 | 87bp | 61°C |
| | Lower 5' CCC TTG GTG GTA TAG GCA GCA 3' (SEQ ID NO: 14) | 15 | | |
| PTPL1 | Upper 5' CAA AGG TGA TCG CGT CCT A 3' (SEQ ID NO: 15) | 26-27 | 148bp | 61°C |
| | Lower 5' CGG GAC ATG TTC TTT AGA TGT T 3' (SEQ ID NO: 16) | 28 | | |
| HPRT | Upper 5' CTG ACC TGC TGG ATT ACA 3' (SEQ ID NO: 17) | 3 | 256bp | 55°C |
| | Lower 5' GCG ACC TTG ACC ATC TTT 3' (SEQ ID NO: 18) | 5 | | |

To confirm the total gene specificity of the sequences chosen for the primers, we performed nucleotide-nucleotide BLASTN against a database of expressed sequence tags and nr (the nonredundant set of the GenBank, EMBL, and DDBJ database sequences). To avoid amplification of contaminating genomic DNA, one of the two primers was placed at the junction between two exons.
The relative quantification of PTP gene expression was performed with the comparative cycle threshold (CT) method where the CT parameter is defined as the cycle number at which the fluorescent signal is first detectable. This method is based on the use of a calibrator sample and an endogenous RNA control, which permits the quantification in the unknown samples. The human breast cancer cell line MCF7, known to express the three PTPs, was chosen as the calibrator sample (PTP expression = 1), and the hypoxanthine phosphoribosyltransferase 1 mRNA as the constant endogenous RNA.
The relative PTP expression was given by the formula: 2- CT, where CT = CT patient sample - CT calibrator sample; with CT = CT PTP - CT HPRT.

**Statistical analyses:** All the statistical analyses were done using the SPSS software (version 11.0). Correlations between parameters were assessed according to the Spearman nonparametric test. Overall survival and relapse-free survival were studied by Kaplan Meier method analysis. Comparison between curves was carried out by the log rank test. The proportional hazard regression method of Cox was used to assess the prognostic significance of parameters taken in association.

### Results :

**Training set:** The classical correlations observed in breast cancer were found between ER and PR (p<0.001, r = 0.761), the histoprognostic grade and ER (p = 0.05, r = - 0.256) or PR (p = 0.045, r = - 0.274), and tumour size and ER (p = 0.05, r = - 0.271). PTPL1 expression, positively correlated with the hormonal receptor status (p=0.02, r=0.307 between PTPL1 and PR).
When looking for the prognostic value the overall survival was significantly longer among patients with a high level of PTPL1 expression than among patients whose tumours had a low level of this protein (P=0.04 by the log rank test).

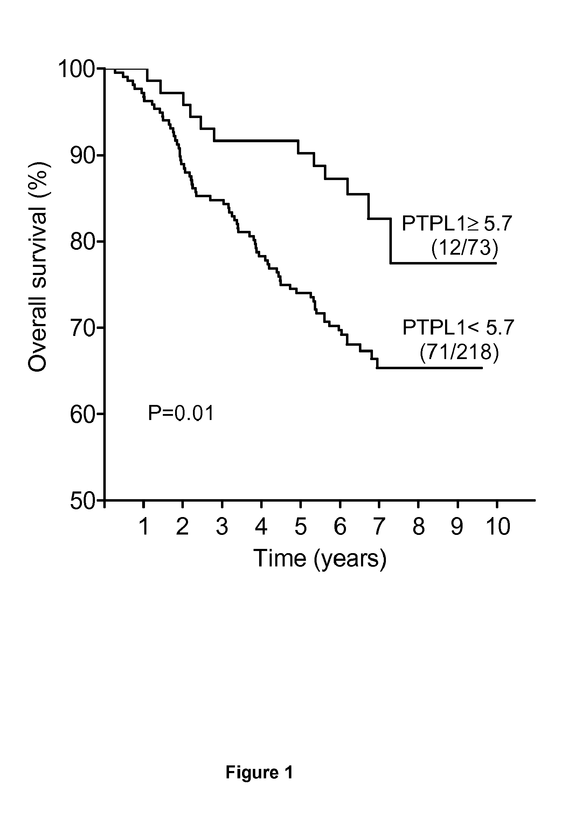

**Testing set:** The PTPL1 expression level, selected during the training set, was then determined in a complementary cohort of 232 breast cancer patients. In the total cohort (291 patients), the classical correlations observed in breast cancer were found between ER and PR (p<0.001, r=0.626), ER and age (p<0.001, r=0.268), and PR and age (p=0.01, r=0.141). Hormone receptors were negatively correlated with histoprognostic grade (p<0.001, r=-0.366 and r=-0.342 for ER and PR respectively) and tumour size (p<0.001, r=-0.229 and p=0.037, r=-0.126 for ER and PR respectively). Node involvement was positively correlated with tumour size (p=0.0015, r=0.19). PTPL1 expression was positively correlated with the hormonal receptor status and negatively with the histoprognostic grade and node invasion.
When looking for the prognostic value of PTPL1, the overall survival was found to be significantly longer among patients with a high level of PTPL1 expression than among patients whose tumours had a low level of this protein (P=0.01 by the log rank test) (Figure 1). In Cox univariate analyses, in addition to PTPL1 expression, as expected, ER and PR expression, tumour size and node invasion were prognostic factors for global survival (Table 3).

**Table 3: Prognostic factors in Cox univariate and multivariate analyses**

| | Total set univariate | | Total set multivariate | |
|---|---|---|---|---|
| | P | RR | P | RR |
| Histoprognostic grade (I, II, III) | 0.23 | | | |
| Node involvement (0;>0) | 0.043 | 1.58 | NS | |
| Tumour size (≤ 2; 2-5; >5 cm) | 0.003 | 1.85 | 0.024 | 1.62 |
| ER (< 10; ≥ 10 fmol/mg protein) | 0.047 | 0.63 | NS | |
| PR (< 10; ≥ 10 fmol/mg protein) | 0.004 | 0.52 | 0.013 | 0.55 |
| PTPL (<5.7; ≥5.7) | 0.012 | 0.45 | 0.035 | 0.48 |

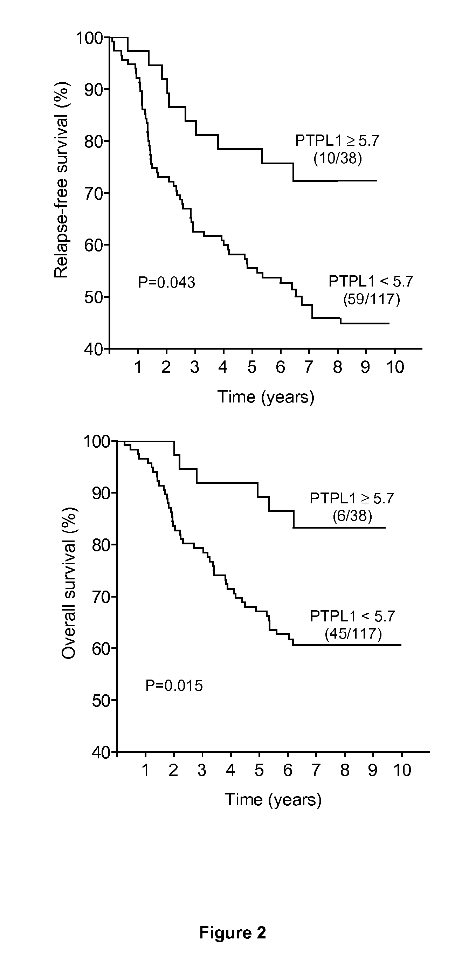

In multivariate analyses, PTPL1 was an independent prognostic factor for overall survival. PR and tumour size preserved their prognostic values and node invasion was no longer a prognostic factor for overall survival (Table 3). In the meantime, only node involvement and tumour size were prognostic factors for relapse free survival in univariate and multivariate analyses.
If we consider the N+ patients (n=155), relapse free and overall survival were significantly longer among patients with high level of PTPL1 expression than among patients whose tumours had low levels of this protein (respectively P=0.015 and p=0.043 by the log rank test) (Figure 2). Accordingly, in Cox univariate analyses PTPL1 was a prognostic factor for relapse free and global survival (P= 0.046, risk ratio = 0.55 and P=0.018, risk ratio = 0.40 respectively), but it did not preserve its prognostic value in multivariate analyses.

### REFERENCES:

Colas P, Cohen B, Jessen T, Grishina I, McCoy J, Brent R. (1996) Genetic selection of peptide aptamers that recognize and inhibit cyclin-dependent kinase 2. Nature, 380, 548-50.
Cote RJ, Morrissey DM, Houghton AN, Beattie EJ Jr, Oettgen HF, Old LJ. Generation of human monoclonal antibodies reactive with cellular antigens. Proc Natl Acad Sci U S A. 1983 Apr;80(7):2026-30.
Esteva FJ, Hortobagyi GN. Prognostic molecular markers in early breast cancer. Breast Cancer Res 2004;6:109-18.
Freiss G, Puech C, Vignon F. Extinction of insulin-like growth factor-I mitogenic signaling by antiestrogen-stimulated Fas-associated protein tyrosine phosphatase-1 in human breast cancer cells. Mol Endocrinol 1998;12:568-79.
Koenders T, Benraad TJ. Quality control of estrogen receptor assays in The Netherlands. Breast Cancer Res Treat 1983;3:255-66.
Kohler G, Milstein C. Continuous cultures of fused cells secreting antibody of predefined specificity. Nature. 1975 Aug 7;256(5517):495-7.
Pawlowski V, Revillion F, Hebbar M, Hornez L, Peyrat JP. Prognostic value of the type I growth factor receptors in a large series of human primary breast cancers quantified with a real-time reverse transcription-polymerase chain reaction assay. Clin Cancer Res 2000;6:4217-25.
Peyrat JP, Vanlemmens L, Fournier J, Huet G, Revillion F, Bonneterre J. Prognostic value of p53 and urokinase-type plasminogen activator in node-negative human breast cancers. Clin Cancer Res 1998;4:189-96.
Thorpe SM, Rochefort H, Garcia M, Freiss G, Christensen IJ, Khalaf S, Paolucci F, Pau B, Rasmussen BB, Rose C. Association between high concentrations of Mr 52,000 cathepsin D and poor prognosis in primary human breast cancer. Cancer Res. 1989 ;49:6008-14.
Tuerk C., Using the SELEX combinatorial chemistry process to find high affinity nucleic acid ligands to target molecules. Methods Mol Biol. 1997; 67: 219-30.

## Claims

1. A method for predicting the survival of a breast cancer patient comprising determining the expression level of the PTPL1 gene in a biological sample obtained from said patient.

2. The method according to claim 1 wherein said biological sample is a cell or tissue sample.

3. The method according to claim 2 wherein said biological sample is a breast biopsy.

4. The method according to any of claims 1 to 3 wherein said expression level of the PTPL1 gene is determined by measuring the quantity of the PTPL1 mRNA in said biological sample obtained from said patient.

5. The method according claim 4, wherein said PTPL1 mRNA is selected in the group comprising SEQ ID NO: 1, SEQ ID NO: 2, SEQ ID NO: 3 and SEQ ID NO:4.

6. The method according to any of claims 1 to 3 said expression level of the PTPL1 gene is determined by measuring the quantity of the PTPL1 protein in said biological sample obtained from said patient.

7. The method according claim 6, wherein said PTPL1 protein is selected in the group comprising SEQ ID NO: 5, SEQ ID NO: 6, SEQ ID NO: 7 and SEQ ID NO:8.


SEQUENCE LISTING

<110>  Institut National Supérieur d'Etude et de Recherche
       Medicale (INSERM)

<120>  PTPL1 AS A BIOMAKER OF SURVIVAL IN BREAST CANCER

<130>  67193

<160>  18

<170>  PatentIn version 3.3

<210>  1
<211>  8119
<212>  DNA
<213>  Homo sapiens

<400>  1
cgtccctgca gccctcgccc ggcgctccag tagcaggacc cggtctcggg accagccggt      60

aatatgcacg tgtcactagc tgaggccctg gaggttcggg gtggaccact tcaggaggaa     120

gaaatatggg ctgtattaaa tcaaagtgct gaaagtctcc aagaattatt cagaaaagta     180

agcctagctg atcctgctgc ccttggcttc atcatttctc catggtctct gctgttgctg     240

ccatctggta gtgtgtcatt tacagatgaa aatatttcca atcaggatct tcgagcattc     300

actgcaccag aggttcttca aaatcagtca ctaacttctc tctcagatgt tgaaaagatc     360

cacatttatt ctcttggaat gacactgtat tggggggctg attatgaagt gcctcagagc     420

caacctatta agcttggaga tcatctcaac agcatactgc ttggaatgtg tgaggatgtt     480

atttacgctc gagtttctgt tcggactgtg ctggatgctt gcagtgccca cattaggaat     540

agcaattgtg caccctcatt ttcctacgtg aaacacttgg taaaactggt tctgggaaat     600

ctttctggga cagatcagct ttcctgtaac agtgaacaaa agcctgatcg aagccaggct     660

attcgagatc gattgcgagg aaaaggatta ccaacaggaa gaagctctac ttctgatgta     720

ctagacatac aaaagcctcc actctctcat cagacctttc ttaacaaagg gcttagtaaa     780

tctatgggat ttctgtccat caaagataca caagatgaga attatttcaa ggacatttta     840

tcagataatt ctggacgtga agattctgaa aatacattct ccccttacca gttcaaaact     900

agtggcccag aaaaaaaacc catccctggc attgatgtgc tttctaagaa gaagatctgg     960

gcttcatcca tggacttgct ttgtacagct gacagagact tctcttcagg agagactgcc    1020

acatatcgtc gttgtcaccc tgaggcagta acagtgcgga cttcaactac tcctagaaaa    1080

aaggaggcaa gatactcaga tggaagtata gccttggata tctttggccc tcagaaaatg    1140

gatccaatat atcacactcg agaattgccc acctcctcag caatatcaag tgctttggac    1200

cgaatccgag agagacaaaa gaaacttcag gttctgaggg aagccatgaa tgtagaagaa    1260

ccagttcgaa gatacaaaac ttatcatggt gatgtcttta gtacctccag tgaaagtcca    1320

tctattattt cctctgaatc agatttcaga caagtgagaa gaagtgaagc ctcaaagagg    1380

tttgaatcca gcagtggtct cccaggggta gatgaaacct taagtcaagg ccagtcacag    1440

agaccgagca gacaatatga aacacccttt gaaggcaact taattaatca agagatcatg    1500

ctaaaacggc aagaggaaga actgatgcag ctacaagcca aaatggccct tagacagtct 1560
cggttgagcc tatatccagg agacacaatc aaagcgtcca tgcttgacat caccagggat 1620
ccgttaagag aaattgccct agaaacagcc atgactcaaa gaaaactgag gaatttcttt 1680
ggccctgagt ttgtgaaaat gacaattgaa ccatttatat ctttggattt gccacggtct 1740
attcttacta agaaagggaa gaatgaggat aaccgaagga aagtaaacat aatgcttctg 1800
aacgggcaaa gactggaact gacctgtgat accaaaacta tatgtaaaga tgtgtttgat 1860
atggttgtgg cacatattgg cttagtagag catcatttgt ttgctttagc taccctcaaa 1920
gataatgaat atttctttgt tgatcctgac ttaaaattaa ccaaagtggc cccagaggga 1980
tggaaagaag aaccaaagaa aaagaccaaa gccactgtta attttacttt gtttttcaga 2040
attaaatttt ttatggatga tgttagtcta atacaacata ctctgacgtg tcatcagtat 2100
taccttcagc ttcgaaaaga tattttggag gaaaggatgc actgtgatga tgagacttcc 2160
ttattgctgg catccttggc tctccaggct gagtatggag attatcaacc agaggttcat 2220
ggtgtgtctt actttagaat ggagcactat ttgcccgcca gagtgatgga gaaacttgat 2280
ttatcctata tcaaagaaga gttacccaaa ttgcataata cctatgtggg agcttctgaa 2340
aaagagacag agttagaatt tttaaaggtc tgccaaagac tgacagaata tggagttcat 2400
tttcaccgag tgcaccctga gaagaagtca caaacaggaa tattgcttgg agtctgttct 2460
aaaggtgtcc ttgtgtttga agttcacaat ggagtgcgca cattggtcct tcgctttcca 2520
tggagggaaa ccaagaaaat atctttttct aaaaagaaaa tcacattgca aaatacatca 2580
gatggaataa aacatggctt ccagacagac aacagtaaga tatgccagta cctgctgcac 2640
ctctgctctt accagcataa gttccagcta cagatgagag caagacagag caaccaagat 2700
gcccaagata ttgagagagc ttcgtttagg agcctgaatc tccaagcaga gtctgttaga 2760
ggatttaata tgggacgagc aatcagcact ggcagtctgg ccagcagcac cctcaacaaa 2820
cttgctgttc gacctttatc agttcaagct gagattctga agaggctatc ctgctcagag 2880
ctgtcgcttt accagccatt gcaaaacagt tcaaaagaga agaatgacaa agcttcatgg 2940
gaggaaaagc ctagagagat gagtaaatca taccatgatc tcagtcaggc ctctctctat 3000
ccacatcgga aaaatgtcat tgttaacatg gaacccccac cacaaaccgt tgcagagttg 3060
gtgggaaaac cttctcacca gatgtcaaga tctgatgcag aatctttggc aggagtgaca 3120
aaacttaata attcaaagtc tgttgcgagt ttaaatagaa gtcctgaaag gaggaaacat 3180
gaatcagact cctcatccat tgaagaccct gggcaagcat atgttctagg aatgactatg 3240
catagttctg gaaactcttc atcccaagta cccttaaaag aaaatgatgt gctacacaaa 3300
agatggagca tagtatcttc accagaaagg gagatcacct tagtgaacct gaaaaaagat 3360
gcaaagtatg gcttgggatt tcaaattatt ggtggggaga agatgggaag actggaccta 3420
ggcatattta tcagttcagt tgcccctgga ggaccagctg acttggatgg atgcttgaag 3480
ccaggagacc gtttgatatc tgtgaatagt gtgagtctgg agggagtcag ccaccatgct 3540

gcaattgaaa ttttgcaaaa tgcacctgaa gatgtgacac ttgttatctc tcagccaaaa 3600
gaaaagatat ccaaagtgcc ttctactcct gtgcatctca ccaatgagat gaaaaactac 3660
atgaagaaat cttcctacat gcaagacagt gctatagatt cttcttccaa ggatcaccac 3720
tggtcacgtg gtaccctgag gcacatctcg gagaactcct ttgggccatc tgggggcctg 3780
cgggaaggaa gcctgagttc tcaagattcc aggactgaga gtgccagctt gtctcaaagc 3840
caggtcaatg gtttctttgc cagccattta ggtgaccaaa cctggcagga atcacagcat 3900
ggcagccctt ccccatctgt aatatccaaa gccaccgaga aagagacttt cactgatagt 3960
aaccaaagca aaactaaaaa gccaggcatt tctgatgtaa ctgattactc agaccgtgga 4020
gattcagaca tggatgaagc cacttactcc agcagtcagg atcatcaaac accaaaacag 4080
gaatcttcct cttcagtgaa tacatccaac aagatgaatt ttaaaacttt ttcttcatca 4140
cctcctaagc ctggagatat ctttgaggtt gaactggcta aaaatgataa cagcttgggg 4200
ataagtgtca cgggaggtgt gaatacgagt gtcagacatg gtggcattta tgtgaaagct 4260
gttattcccc agggagcagc agagtctgat ggtagaattc acaaaggtga tcgcgtccta 4320
gctgtcaatg gagttagtct agaaggagcc acccataagc aagctgtgga aacactgaga 4380
aatacaggac aggtggttca tctgttatta gaaaagggac aatctccaac atctaaagaa 4440
catgtcccgg taaccccaca gtgtaccctt tcagatcaga atgcccaagg tcaaggccca 4500
gaaaaagtga agaaaacaac tcaggtcaaa gactacagct ttgtcactga agaaaataca 4560
tttgaggtaa aattatttaa aaatagctca ggtctaggat tcagtttttc tcgagaagat 4620
aatcttatac cggagcaaat taatgccagc atagtaaggg ttaaaaagct ctttcctgga 4680
cagccagcag cagaaagtgg aaaaattgat gtaggagatg ttatcttgaa agtgaatgga 4740
gcctctttga aaggactatc tcagcaggaa gtcatatctg ctctcagggg aactgctcca 4800
gaagtattct tgcttctctg cagacctcca cctggtgtgc taccggaaat tgatactgcg 4860
cttttgaccc cacttcagtc tccagcacaa gtacttccaa acagcagtaa agactcttct 4920
cagccatcat gtgtggagca aagcaccagc tcagatgaaa atgaaatgtc agacaaaagc 4980
aaaaaacagt gcaagtcccc atccagaaga gacagttaca gtgacagcag tgggagtgga 5040
gaagatgact tagtgacagc tccagcaaac atatcaaatt cgacctggag ttcagctttg 5100
catcagactc taagcaacat ggtatcacag gcacagagtc atcatgaagc acccaagagt 5160
caagaagata ccatttgtac catgttttac tatcctcaga aaattcccaa taaaccagag 5220
tttgaggaca gtaatccttc ccctctacca ccggatatgg ctcctgggca gagttatcaa 5280
ccccaatcag aatctgcttc ctctagttcg atggataagt atcatataca tcacatttct 5340
gaaccaacta gacaagaaaa ctggacacct ttgaaaaatg acttggaaaa tcaccttgaa 5400
gactttgaac tggaagtaga actcctcatt accctaatta aatcagaaaa aggaagcctg 5460
ggttttacag taaccaaagg caatcagaga attggttgtt atgttcatga tgtcatacag 5520
gatccagcca aaagtgatgg aaggctaaaa cctggggacc ggctcataaa ggttaatgat 5580

acagatgtta ctaatatgac tcatacagat gcagttaatc tgctccgggc tgcatccaaa 5640
acagtcagat tagttattgg acgagttcta gaattaccca gaataccaat gttgcctcat 5700
ttgctaccgg acataacact aacgtgcaac aaagaggagt tgggtttttc cttatgtgga 5760
ggtcatgaca gcctttatca agtggtatat attagtgata ttaatccaag gtccgtcgca 5820
gccattgagg gtaatctcca gctattagat gtcatccatt atgtgaacgg agtcagcaca 5880
caaggaatga ccttggagga agttaacaga gcattagaca tgtcacttcc ttcattggta 5940
ttgaaagcaa caagaaatga tcttccagtg gtccccagct caaagaggtc tgctgtttca 6000
gctccaaagt caaccaaagg caatggttcc tacagtgtgg ggtcttgcag ccagcctgcc 6060
ctcactccta atgattcatt ctccacggtt gctggggaag aaataaatga aatatcgtac 6120
cccaaaggaa aatgttctac ttatcagata aagggatcac caaacttgac tctgcccaaa 6180
gaatcttata tacaagaaga tgacatttat gatgattccc aagaagctga agttatccag 6240
tctctgctgg atgttgtgga tgaggaagcc cagaatcttt taaacgaaaa taatgcagca 6300
ggatactcct gtggtccagg tacattaaag atgaatggga agttatcaga agagagaaca 6360
gaagatacag actgcgatgg ttcaccttta cctgagtatt ttactgaggc caccaaaatg 6420
aatggctgtg aagaatattg tgaagaaaaa gtaaaaagtg aaagcttaat tcagaagcca 6480
caagaaaaga agactgatga tgatgaaata acatggggaa atgatgagtt gccaatagag 6540
agaacaaacc atgaagattc tgataaagat cattcctttc tgacaaacga tgagctcgct 6600
gtactccctg tcgtcaaagt gcttccctct ggtaaataca cgggtgccaa cttaaaatca 6660
gtcattcgag tcctgcgggg tttgctagat caaggaattc cttctaagga gctggagaat 6720
cttcaagaat taaaaccttt ggatcagtgt ctaattgggc aaactaagga aaacagaagg 6780
aagaacagat ataaaaatat acttccctat gatgctacaa gagtgcctct tggagatgaa 6840
ggtggctata tcaatgccag cttcattaag ataccagttg ggaaagaaga gttcgtttac 6900
attgcctgcc aaggaccact gcctacaact gttggagact tctggcagat gatttgggag 6960
caaaaatcca cagtgatagc catgatgact caagaagtag aaggagaaaa aatcaaatgc 7020
cagcgctatt ggcccaacat cctaggcaaa acaacaatgg tcagcaacag acttcgactg 7080
gctcttgtga gaatgcagca gctgaagggc tttgtggtga gggcaatgac ccttgaagat 7140
attcagacca gagaggtgcg ccatatttct catctgaatt tcactgcctg gccagaccat 7200
gatacacctt ctcaaccaga tgatctgctt acttttatct cctacatgag acacatccac 7260
agatcaggcc caatcattac gcactgcagt gctggcattg gacgttcagg gaccctgatt 7320
tgcatagatg tggttctggg attaatcagt caggatcttg attttgacat ctctgatttg 7380
gtgcgctgca tgagactaca aagacacgga atggttcaga cagaggatca atatattttc 7440
tgctatcaag tcatccttta tgtcctgaca cgtcttcaag cagaagaaga gcaaaaacag 7500
cagcctcagc ttctgaagtg acatgaaaag agcctctgga tgcatttcca tttctctcct 7560
taacctccag cagactcctg ctctctatcc aaaataaaga tcacagagca gcaagttcat 7620

acaacatgca tgttctcctc tatcttagag gggtattctt cttgaaaata aaaaatattg 7680

aaatgctgta tttttacagc tactttaacc tatgataatt atttacaaaa ttttaacact 7740

aaccaaacaa tgcagatctt agggatgatt aaaggcagca tttgatgata gcagacattg 7800

ttacaaggac atggtgagtc tatttttaat gcaccaatct tgtttatagc aaaaatgttt 7860

tccaatattt taataaagta gttattttat aggggatact tgaaaccagt atttaagctt 7920

taaatgacag taatattggc atagaaaaaa gtagcaaatg tttactgtat caatttctaa 7980

tgtttactat atagaatttc ctgtaatata tttatatact ttttcatgaa aatggagtta 8040

tcagttatct gtttgttact gcatcatctg tttgtaatca ttatctcact ttgtaaataa 8100

aaacacacct taaaacatg 8119

<210> 2
<211> 7546
<212> DNA
<213> Homo sapiens

<400> 2
cgtccctgca gccctcgccc ggcgctccag tagcaggacc cggtctcggg accagccggt 60

aatatgcacg tgtcactagc tgaggccctg gaggttcggg gtggaccact tcaggaggaa 120

gaaatatggg ctgtattaaa tcaaagtgct gaaagtctcc aagaattatt cagaaaagta 180

agcctagctg atcctgctgc ccttggcttc atcatttctc catggtctct gctgttgctg 240

ccatctggta gtgtgtcatt tacagatgaa aatatttcca atcaggatct tcgagcattc 300

actgcaccag aggttcttca aaatcagtca ctaacttctc tctcagatgt tgaaaagatc 360

cacatttatt ctcttggaat gacactgtat tggggggctg attatgaagt gcctcagagc 420

caacctatta agcttggaga tcatctcaac agcatactgc ttggaatgtg tgaggatgtt 480

atttacgctc gagtttctgt tcggactgtg ctggatgctt gcagtgccca cattaggaat 540

agcaattgtg caccctcatt ttcctacgtg aaacacttgg taaaactggt tctgggaaat 600

ctttctggga cagatcagct ttcctgtaac agtgaacaaa agcctgatcg aagccaggct 660

attcgagatc gattgcgagg aaaaggatta ccaacaggaa gaagctctac ttctgatgta 720

ctagacatac aaaagcctcc actctctcat cagacctttc ttaacaaagg gcttagtaaa 780

tctatgggat ttctgtccat caaagataca caagatgaga attatttcaa ggacatttta 840

tcagataatt ctggacgtga agattctgaa aatacattct ccccttacca gttcaaaact 900

agtggcccag aaaaaaaacc catccctggc attgatgtgc tttctaagaa gaagatctgg 960

gcttcatcca tggacttgct ttgtacagct gacagagact tctcttcagg agagactgcc 1020

acatatcgtc gttgtcaccc tgaggcagta acagtgcgga cttcaactac tcctagaaaa 1080

aaggaggcaa gatactcaga tggaagtata gccttggata tctttggccc tcagaaaatg 1140

gatccaatat atcacactcg agaattgccc acctcctcag caatatcaag tgctttggac 1200

cgaatccgag agagacaaaa gaaacttcag gttctgaggg aagccatgaa tgtagaagaa 1260

ccagttcgaa gatacaaaac ttatcatggt gatgtcttta gtacctccag tgaaagtcca 1320
tctattattt cctctgaatc agatttcaga caagtgagaa gaagtgaagc ctcaaagagg 1380
tttgaatcca gcagtggtct cccaggggta gatgaaacct taagtcaagg ccagtcacag 1440
agaccgagca gacaatatga aacacccttt gaaggcaact taattaatca agagatcatg 1500
ctaaaacggc aagaggaaga actgatgcag ctacaagcca aaatggccct tagacagtct 1560
cggttgagcc tatatccagg agacacaatc aaagcgtcca tgcttgacat caccagggat 1620
ccgttaagag aaattgccct agaaacagcc atgactcaaa gaaaactgag gaatttcttt 1680
ggccctgagt ttgtgaaaat gacaattgaa ccatttatat ctttggattt gccacggtct 1740
attcttacta agaaagggaa gaatgaggat aaccgaagga aagtaaacat aatgcttctg 1800
aacgggcaaa gactggaact gacctgtgat accaaaacta tatgtaaaga tgtgtttgat 1860
atggttgtgg cacatattgg cttagtagag catcatttgt ttgctttagc taccctcaaa 1920
gataatgaat atttctttgt tgatcctgac ttaaaattaa ccaaagtggc cccagaggga 1980
tggaaagaag aaccaaagaa aaagaccaaa gccactgtta attttacttt gtttttcaga 2040
attaaatttt ttatggatga tgttagtcta atacaacata ctctgacgtg tcatcagtat 2100
taccttcagc ttcgaaaaga tattttggag gaaaggatgc actgtgatga tgagacttcc 2160
ttattgctgg catccttggc tctccaggct gagtatggag attatcaacc agaggttcat 2220
ggtgtgtctt actttagaat ggagcactat ttgcccgcca gagtgatgga gaaacttgat 2280
ttatcctata tcaaagaaga gttacccaaa ttgcataata cctatgtggg agcttctgaa 2340
aaagagacag agttagaatt tttaaaggtc tgccaaagac tgacagaata tggagttcat 2400
tttcaccgag tgcaccctga gaagaagtca caaacaggaa tattgcttgg agtctgttct 2460
aaaggtgtcc ttgtgtttga agttcacaat ggagtgcgca cattggtcct tcgctttcca 2520
tggagggaaa ccaagaaaat atctttttct aaaaagaaaa tcacattgca aaatacatca 2580
gatggaataa aacatggctt ccagacagac aacagtaaga tatgccagta cctgctgcac 2640
ctctgctctt accagcataa gttccagcta cagatgagag caagacagag caaccaagat 2700
gcccaagata ttgatgtgct acacaaaaga tggagcatag tatcttcacc agaaagggag 2760
atcaccttag tgaacctgaa aaaagatgca aagtatggct tgggatttca aattattggt 2820
ggggagaaga tgggaagact ggacctaggc atatttatca gttcagttgc ccctggagga 2880
ccagctgact tggatggatg cttgaagcca ggagaccgtt tgatatctgt gaatagtgtg 2940
agtctggagg gagtcagcca ccatgctgca attgaaattt tgcaaaatgc acctgaagat 3000
gtgacacttg ttatctctca gccaaaagaa aagatatcca aagtgccttc tactcctgtg 3060
catctcacca atgagatgaa aaactacatg aagaaatctt cctacatgca agacagtgct 3120
atagattctt cttccaagga tcaccactgg tcacgtggta ccctgaggca catctcggag 3180
aactcctttg ggccatctgg gggcctgcgg gaaggaagcc tgagttctca agattccagg 3240
actgagagtg ccagcttgtc tcaaagccag gtcaatggtt tctttgccag ccatttaggt 3300

gaccaaacct ggcaggaatc acagcatggc agcccttccc catctgtaat atccaaagcc 3360
accgagaaag agactttcac tgatagtaac caaagcaaaa ctaaaaagcc aggcatttct 3420
gatgtaactg attactcaga ccgtggagat tcagacatgg atgaagccac ttactccagc 3480
agtcaggatc atcaaacacc aaaacaggaa tcttcctctt cagtgaatac atccaacaag 3540
atgaatttta aaactttttc ttcatcacct cctaagcctg gagatatctt tgaggttgaa 3600
ctggctaaaa atgataacag cttggggata agtgtcacgg gaggtgtgaa tacgagtgtc 3660
agacatggtg gcatttatgt gaaagctgtt attccccagg gagcagcaga gtctgatggt 3720
agaattcaca aaggtgatcg cgtcctagct gtcaatggag ttagtctaga aggagccacc 3780
cataagcaag ctgtggaaac actgagaaat acaggacagg tggttcatct gttattagaa 3840
aagggacaat ctccaacatc taaagaacat gtcccggtaa ccccacagtg taccctttca 3900
gatcagaatg cccaaggtca aggcccagaa aaagtgaaga aaacaactca ggtcaaagac 3960
tacagctttg tcactgaaga aaatacattt gaggtaaaat tatttaaaaa tagctcaggt 4020
ctaggattca gtttttctcg agaagataat cttataccgg agcaaattaa tgccagcata 4080
gtaagggtta aaaagctctt tcctggacag ccagcagcag aaagtggaaa aattgatgta 4140
ggagatgtta tcttgaaagt gaatggagcc tctttgaaag gactatctca gcaggaagtc 4200
atatctgctc tcaggggaac tgctccagaa gtattcttgc ttctctgcag acctccacct 4260
ggtgtgctac cggaaattga tactgcgctt ttgaccccac ttcagtctcc agcacaagta 4320
cttccaaaca gcagtaaaga ctcttctcag ccatcatgtg tggagcaaag caccagctca 4380
gatgaaaatg aaatgtcaga caaaagcaaa aaacagtgca agtccccatc cagaagagac 4440
agttacagtg acagcagtgg gagtggagaa gatgacttag tgacagctcc agcaaacata 4500
tcaaattcga cctggagttc agctttgcat cagactctaa gcaacatggt atcacaggca 4560
cagagtcatc atgaagcacc caagagtcaa gaagatacca tttgtaccat gttttactat 4620
cctcagaaaa ttcccaataa accagagttt gaggacagta atccttcccc tctaccaccg 4680
gatatggctc ctgggcagag ttatcaaccc caatcagaat ctgcttcctc tagttcgatg 4740
gataagtatc atatacatca catttctgaa ccaactagac aagaaaactg gacacctttg 4800
aaaaatgact tggaaaatca ccttgaagac tttgaactgg aagtagaact cctcattacc 4860
ctaattaaat cagaaaaagg aagcctgggt tttacagtaa ccaaaggcaa tcagagaatt 4920
ggttgttatg ttcatgatgt catacaggat ccagccaaaa gtgatggaag gctaaaacct 4980
ggggaccggc tcataaaggt taatgataca gatgttacta atatgactca tacagatgca 5040
gttaatctgc tccgggctgc atccaaaaca gtcagattag ttattggacg agttctagaa 5100
ttacccagaa taccaatgtt gcctcatttg ctaccggaca taacactaac gtgcaacaaa 5160
gaggagttgg gtttttcctt atgtggaggt catgacagcc tttatcaagt ggtatatatt 5220
agtgatatta atccaaggtc cgtcgcagcc attgagggta atctccagct attagatgtc 5280
atccattatg tgaacggagt cagcacacaa ggaatgacct tggaggaagt taacagagca 5340

ttagacatgt cacttccttc attggtattg aaagcaacaa gaaatgatct tccagtggtc 5400
cccagctcaa agaggtctgc tgtttcagct ccaaagtcaa ccaaaggcaa tggttcctac 5460
agtgtggggt cttgcagcca gcctgccctc actcctaatg attcattctc cacggttgct 5520
ggggaagaaa taaatgaaat atcgtacccc aaaggaaaat gttctactta tcagataaag 5580
ggatcaccaa acttgactct gcccaaagaa tcttatatac aagaagatga catttatgat 5640
gattcccaag aagctgaagt tatccagtct ctgctggatg ttgtggatga ggaagcccag 5700
aatcttttaa acgaaaataa tgcagcagga tactcctgtg gtccaggtac attaaagatg 5760
aatgggaagt tatcagaaga gagaacagaa gatacagact gcgatggttc acctttacct 5820
gagtatttta ctgaggccac caaaatgaat ggctgtgaag aatattgtga agaaaaagta 5880
aaaagtgaaa gcttaattca gaagccacaa gaaaagaaga ctgatgatga tgaaataaca 5940
tggggaaatg atgagttgcc aatagagaga acaaaccatg aagattctga taaagatcat 6000
tcctttctga caaacgatga gctcgctgta ctccctgtcg tcaaagtgct tccctctggt 6060
aaatacacgg gtgccaactt aaaatcagtc attcgagtcc tgcggggttt gctagatcaa 6120
ggaattcctt ctaaggagct ggagaatctt caagaattaa aacctttgga tcagtgtcta 6180
attgggcaaa ctaaggaaaa cagaaggaag aacagatata aaaatatact tccctatgat 6240
gctacaagag tgcctcttgg agatgaaggt ggctatatca atgccagctt cattaagata 6300
ccagttggga aagaagagtt cgtttacatt gcctgccaag gaccactgcc tacaactgtt 6360
ggagacttct ggcagatgat ttgggagcaa aaatccacag tgatagccat gatgactcaa 6420
gaagtagaag gagaaaaaat caaatgccag cgctattggc ccaacatcct aggcaaaaca 6480
acaatggtca gcaacagact tcgactggct cttgtgagaa tgcagcagct gaagggcttt 6540
gtggtgaggg caatgaccct tgaagatatt cagaccagag aggtgcgcca tatttctcat 6600
ctgaatttca ctgcctggcc agaccatgat acaccttctc aaccagatga tctgcttact 6660
tttatctcct acatgagaca catccacaga tcaggcccaa tcattacgca ctgcagtgct 6720
ggcattggac gttcagggac cctgatttgc atagatgtgg ttctgggatt aatcagtcag 6780
gatcttgatt ttgacatctc tgatttggtg cgctgcatga gactacaaag acacggaatg 6840
gttcagacag aggatcaata tattttctgc tatcaagtca tcctttatgt cctgacacgt 6900
cttcaagcag aagaagagca aaaacagcag cctcagcttc tgaagtgaca tgaaaagagc 6960
ctctggatgc atttccattt ctctccttaa cctccagcag actcctgctc tctatccaaa 7020
ataaagatca cagagcagca agttcataca acatgcatgt tctcctctat cttagagggg 7080
tattcttctt gaaaataaaa aatattgaaa tgctgtattt ttacagctac tttaacctat 7140
gataattatt tacaaaattt taacactaac caaacaatgc agatcttagg gatgattaaa 7200
ggcagcattt gatgatagca gacattgtta caaggacatg gtgagtctat ttttaatgca 7260
ccaatcttgt ttatagcaaa aatgttttcc aatattttaa taaagtagtt attttatagg 7320
ggatacttga aaccagtatt taagctttaa atgacagtaa tattggcata gaaaaaagta 7380

gcaaatgttt actgtatcaa tttctaatgt ttactatata gaatttcctg taatatattt 7440

atatactttt tcatgaaaat ggagttatca gttatctgtt tgttactgca tcatctgttt 7500

gtaatcatta tctcactttg taaataaaaa cacaccttaa aacatg 7546

<210> 3
<211> 8133
<212> DNA
<213> Homo sapiens

<400> 3
cgtccctgca gccctcgccc ggcgctccag tagcaggacc cggtctcggg accagccggt 60

aatatgcacg tgtcactagc tgaggccctg gaggttcggg gtggaccact tcaggaggaa 120

gaaatatggg ctgtattaaa tcaaagtgct gaaagtctcc aagaattatt cagaaaagta 180

agcctagctg atcctgctgc ccttggcttc atcatttctc catggtctct gctgttgctg 240

ccatctggta gtgtgtcatt tacagatgaa aatatttcca atcaggatct tcgagcattc 300

actgcaccag aggttcttca aaatcagtca ctaacttctc tctcagatgt tgaaaagatc 360

cacatttatt ctcttggaat gacactgtat tggggggctg attatgaagt gcctcagagc 420

caacctatta agcttggaga tcatctcaac agcatactgc ttggaatgtg tgaggatgtt 480

atttacgctc gagtttctgt tcggactgtg ctggatgctt gcagtgccca cattaggaat 540

agcaattgtg caccctcatt ttcctacgtg aaacacttgg taaaactggt tctgggaaat 600

ctttctggga cagatcagct ttcctgtaac agtgaacaaa agcctgatcg aagccaggct 660

attcgagatc gattgcgagg aaaaggatta ccaacaggaa gaagctctac ttctgatgta 720

ctagacatac aaaagcctcc actctctcat cagacctttc ttaacaaagg gcttagtaaa 780

tctatgggat ttctgtccat caaagataca caagatgaga attatttcaa ggacatttta 840

tcagataatt ctggacgtga agattctgaa aatacattct ccccttacca gttcaaaact 900

agtggcccag aaaaaaaacc catccctggc attgatgtgc tttctaagaa gaagatctgg 960

gcttcatcca tggacttgct ttgtacagct gacagagact tctcttcagg agagactgcc 1020

acatatcgtc gttgtcaccc tgaggcagta acagtgcgga cttcaactac tcctagaaaa 1080

aaggaggcaa gatactcaga tggaagtata gccttggata tctttggccc tcagaaaatg 1140

gatccaatat atcacactcg agaattgccc acctcctcag caatatcaag tgctttggac 1200

cgaatccgag agagacaaaa gaaacttcag gttctgaggg aagccatgaa tgtagaagaa 1260

ccagttcgaa gatacaaaac ttatcatggt gatgtcttta gtacctccag tgaaagtcca 1320

tctattattt cctctgaatc agatttcaga caagtgagaa gaagtgaagc ctcaaagagg 1380

tttgaatcca gcagtggtct cccaggggta gatgaaacct taagtcaagg ccagtcacag 1440

agaccgagca gacaatatga aacacccttt gaaggcaact taattaatca agagatcatg 1500

ctaaaacggc aagaggaaga actgatgcag ctacaagcca aaatggccct tagacagtct 1560

cggttgagcc tatatccagg agacacaatc aaagcgtcca tgcttgacat caccagggat 1620

ccgttaagag aaattgccct agaaacagcc atgactcaaa gaaaactgag gaatttcttt 1680

ggccctgagt ttgtgaaaat gacaattgaa ccatttatat ctttggattt gccacggtct 1740
attcttacta agaaagggaa gaatgaggat aaccgaagga aagtaaacat aatgcttctg 1800
aacgggcaaa gactggaact gacctgtgat accaaaacta tatgtaaaga tgtgtttgat 1860
atggttgtgg cacatattgg cttagtagag catcatttgt ttgctttagc taccctcaaa 1920
gataatgaat atttctttgt tgatcctgac ttaaaattaa ccaaagtggc cccagaggga 1980
tggaaagaag aaccaaagaa aaagaccaaa gccactgtta attttacttt gtttttcaga 2040
attaaatttt ttatggatga tgttagtcta atacaacata ctctgacgtg tcatcagtat 2100
taccttcagc ttcgaaaaga tattttggag gaaaggatgc actgtgatga tgagacttcc 2160
ttattgctgg catccttggc tctccaggct gagtatggag attatcaacc agaggttcat 2220
ggtgtgtctt actttagaat ggagcactat ttgcccgcca gagtgatgga gaaacttgat 2280
ttatcctata tcaaagaaga gttacccaaa ttgcataata cctatgtggg agcttctgaa 2340
aaagagacag agttagaatt tttaaaggtc tgccaaagac tgacagaata tggagttcat 2400
tttcaccgag tgcaccctga gaagaagtca caaacaggaa tattgcttgg agtctgttct 2460
aaaggtgtcc ttgtgtttga agttcacaat ggagtgcgca cattggtcct tcgctttcca 2520
tggagggaaa ccaagaaaat atctttttct aaaaagaaaa tcacattgca aaatacatca 2580
gatggaataa aacatggctt ccagacagac aacagtaaga tatgccagta cctgctgcac 2640
ctctgctctt accagcataa gttccagcta cagatgagag caagacagag caaccaagat 2700
gcccaagata ttgagagagc ttcgtttagg agcctgaatc tccaagcaga gtctgttaga 2760
ggatttaata tgggacgagc aatcagcact ggcagtctgg ccagcagcac cctcaacaaa 2820
cttgctgttc gacctttatc agttcaagct gagattctga agaggctatc ctgctcagag 2880
ctgtcgcttt accagccatt gcaaaacagt tcaaaagaga agaatgacaa agcttcatgg 2940
gaggaaaagc ctagagagat gagtaaatca taccatgatc tcagtcaggc ctctctctat 3000
ccacatcgga aaaatgtcat tgttaacatg gaacccccac cacaaaccgt tgcagagttg 3060
gtgggaaaac cttctcacca gatgtcaaga tctgatgcag aatctttggc aggagtgaca 3120
aaacttaata attcaaagtc tgttgcgagt ttaaatagaa gtcctgaaag gaggaaacat 3180
gaatcagact cctcatccat tgaagaccct gggcaagcat atgttctagg aatgactatg 3240
catagttctg gaaactcttc atcccaagta cccttaaaag aaaatgatgt gctacacaaa 3300
agatggagca tagtatcttc accagaaagg gagatcacct tagtgaacct gaaaaaagat 3360
gcaaagtatg gcttgggatt tcaaattatt ggtggggaga agatgggaag actggaccta 3420
ggcatattta tcagttcagt tgcccctgga ggaccagctg acttggatgg atgcttgaag 3480
ccaggagacc gtttgatatc tgtgaatagt gtgagtctgg agggagtcag ccaccatgct 3540
gcaattgaaa ttttgcaaaa tgcacctgaa gatgtgacac ttgttatctc tcagccaaaa 3600
gaaaagatat ccaaagtgcc ttctactcct gtgcatctca ccaatgagat gaaaaactac 3660
atgaagaaat cttcctacat gcaagacagt gctatagatt cttcttccaa ggatcaccac 3720

tggtcacgtg gtaccctgag gcacatctcg gagaactcct ttgggccgtc tgggggcctg 3780
cgggaaggaa gcctgagttc tcaagattcc aggactgaga gtgccagctt gtctcaaagc 3840
caggtcaatg gtttctttgc cagccattta ggtgaccaaa cctggcagga atcacagcat 3900
ggcagccctt ccccatctgt aatatccaaa gccaccgaga aagagacttt cactgatagt 3960
aaccaaagca aaactaaaaa gccaggcatt tctgatgtaa ctgattactc agaccgtgga 4020
gattcagaca tggatgaagc cacttactcc agcagtcagg atcatcaaac accaaaacag 4080
gaatcttcct cttcagtgaa tacatccaac aagatgaatt ttaaaacttt ttcttcatca 4140
cctcctaagc ctggagatat ctttgaggtt gaactggcta aaaatgataa cagcttgggg 4200
ataagtgtca cggtactgtt tgacaaggga ggtgtgaata cgagtgtcag acatggtggc 4260
atttatgtga aagctgttat tccccaggga gcagcagagt ctgatggtag aattcacaaa 4320
ggtgatcgcg tcctagctgt caatggagtt agtctagaag gagccaccca taagcaagct 4380
gtggaaacac tgagaaatac aggacaggtg gttcatctgt tattagaaaa gggacaatct 4440
ccaacatcta aagaacatgt cccggtaacc ccacagtgta ccctttcaga tcagaatgcc 4500
caaggtcaag gcccagaaaa agtgaagaaa acaactcagg tcaaagacta cagctttgtc 4560
actgaagaaa atacatttga ggtaaaatta tttaaaaata gctcaggtct aggattcagt 4620
ttttctcgag aagataatct tataccggag caaattaatg ccagcatagt aagggttaaa 4680
aagctctttc ctggacagcc agcagcagaa agtggaaaaa ttgatgtagg agatgttatc 4740
ttgaaagtga atggagcctc tttgaaagga ctatctcagc aggaagtcat atctgctctc 4800
aggggaactg ctccagaagt attcttgctt ctctgcagac ctccacctgg tgtgctaccg 4860
gaaattgata ctgcgctttt gaccccactt cagtctccag cacaagtact tccaaacagc 4920
agtaaagact cttctcagcc atcatgtgtg gagcaaagca ccagctcaga tgaaaatgaa 4980
atgtcagaca aaagcaaaaa acagtgcaag tccccatcca gaagagacag ttacagtgac 5040
agcagtggga gtggagaaga tgacttagtg acagctccag caaacatatc aaattcgacc 5100
tggagttcag ctttgcatca gactctaagc aacatggtat cacaggcaca gagtcatcat 5160
gaagcaccca agagtcaaga agataccatt tgtaccatgt tttactatcc tcagaaaatt 5220
cccaataaac cagagtttga ggacagtaat ccttcccctc taccaccgga tatggctcct 5280
gggcagagtt atcaacccca atcagaatct gcttcctcta gttcgatgga taagtatcat 5340
atacatcaca tttctgaacc aactagacaa gaaaactgga cacctttgaa aaatgacttg 5400
gaaaatcacc ttgaagactt tgaactggaa gtagaactcc tcattaccct aattaaatca 5460
gaaaaaggaa gcctgggttt tacagtaacc aaaggcaatc agagaattgg ttgttatgtt 5520
catgatgtca tacaggatcc agccaaaagt gatggaaggc taaaacctgg ggaccggctc 5580
ataaaggtta atgatacaga tgttactaat atgactcata cagatgcagt taatctgctc 5640
cgggctgcat ccaaaacagt cagattagtt attggacgag ttctagaatt acccagaata 5700
ccaatgttgc ctcatttgct accggacata acactaacgt gcaacaaaga ggagttgggt 5760

ttttccttat gtggaggtca tgacagcctt tatcaagtgg tatatattag tgatattaat 5820
ccaaggtccg tcgcagccat tgagggtaat ctccagctat tagatgtcat ccattatgtg 5880
aacggagtca gcacacaagg aatgaccttg gaggaagtta acagagcatt agacatgtca 5940
cttccttcat tggtattgaa agcaacaaga aatgatcttc cagtggtccc cagctcaaag 6000
aggtctgctg tttcagctcc aaagtcaacc aaaggcaatg gttcctacag tgtggggtct 6060
tgcagccagc ctgccctcac tcctaatgat tcattctcca cggttgctgg ggaagaaata 6120
aatgaaatat cgtaccccaa aggaaaatgt tctacttatc agataaaggg atcaccaaac 6180
ttgactctgc ccaaagaatc ttatatacaa gaagatgaca tttatgatga ttcccaagaa 6240
gctgaagtta tccagtctct gctggatgtt gtggatgagg aagcccagaa tcttttaaac 6300
gaaaataatg cagcaggata ctcctgtggt ccaggtacat taaagatgaa tgggaagtta 6360
tcagaagaga gaacagaaga tacagactgc gatggttcac ctttacctga gtattttact 6420
gaggccacca aaatgaatgg ctgtgaagaa tattgtgaag aaaaagtaaa aagtgaaagc 6480
ttaattcaga agccacaaga aaagaagact gatgatgatg aaataacatg gggaaatgat 6540
gagttgccaa tagagagaac aaaccatgaa gattctgata aagatcattc ctttctgaca 6600
aacgatgagc tcgctgtact ccctgtcgtc aaagtgcttc cctctggtaa atacacgggt 6660
gccaacttaa aatcagtcat tcgagtcctg cggggtttgc tagatcaagg aattccttct 6720
aaggagctgg agaatcttca agaattaaaa cctttggatc agtgtctaat tgggcaaact 6780
aaggaaaaca gaaggaagaa cagatataaa aatatacttc cctatgatgc tacaagagtg 6840
cctcttggag atgaaggtgg ctatatcaat gccagcttca ttaagatacc agttgggaaa 6900
gaagagttcg tttacattgc ctgccaagga ccactgccta caactgttgg agacttctgg 6960
cagatgattt gggagcaaaa atccacagtg atagccatga tgactcaaga agtagaagga 7020
gaaaaaatca aatgccagcg ctattggccc aacatcctag gcaaaacaac aatggtcagc 7080
aacagacttc gactggctct tgtgagaatg cagcagctga agggctttgt ggtgagggca 7140
atgacccttg aagatattca gaccagagag gtgcgccata tttctcatct gaatttcact 7200
gcctggccag accatgatac accttctcaa ccagatgatc tgcttacttt tatctcctac 7260
atgagacaca tccacagatc aggcccaatc attacgcact gcagtgctgg cattggacgt 7320
tcagggaccc tgatttgcat agatgtggtt ctgggattaa tcagtcagga tcttgatttt 7380
gacatctctg atttggtgcg ctgcatgaga ctacaaagac acggaatggt tcagacagag 7440
gatcaatata ttttctgcta tcaagtcatc ctttatgtcc tgacacgtct tcaagcagaa 7500
gaagagcaaa aacagcagcc tcagcttctg aagtgacatg aaaagagcct ctggatgcat 7560
ttccatttct ctccttaacc tccagcagac tcctgctctc tatccaaaat aagatcacag 7620
agcagcaagt tcatacaaca tgcatgttct cctctatctt agaggggtat tcttcttgaa 7680
aataaaaaat attgaaatgc tgtattttta cagctacttt aacctatgat aattatttac 7740
aaaattttaa cactaaccaa acaatgcaga tcttagggat gattaaaggc agcatttgat 7800

gatagcagac attgttacaa ggacatggtg agtctatttt taatgcacca atcttgttta 7860

tagcaaaaat gttttccaat attttaataa agtagttatt ttatagggga tacttgaaac 7920

cagtatttaa gctttaaatg acagtaatat tggcatagaa aaaagtagca aatgtttact 7980

gtatcaattt ctaatgttta ctatatagaa tttcctgtaa tatatttata tactttttca 8040

tgaaaatgga gttatcagtt atctgtttgt tactgcatca tctgtttgta atcattatct 8100

cactttgtaa ataaaaacac accttaaaac atg 8133

<210> 4
<211> 8062
<212> DNA
<213> Homo sapiens

<400> 4
cgtccctgca gccctcgccc ggcgctccag tagcaggacc cggtctcggg accagccggt 60

aatatgcacg tgtcactagc tgaggccctg gaggttcggg gtggaccact tcaggaggaa 120

gaaatatggg ctgtattaaa tcaaagtgct gaaagtctcc aagaattatt cagaaaagta 180

agcctagctg atcctgctgc ccttggcttc atcatttctc catggtctct gctgttgctg 240

ccatctggta gtgtgtcatt tacagatgaa aatatttcca atcaggatct tcgagcattc 300

actgcaccag aggttcttca aaatcagtca ctaacttctc tctcagatgt tgaaaagatc 360

cacatttatt ctcttggaat gacactgtat tggggggctg attatgaagt gcctcagagc 420

caacctatta agcttggaga tcatctcaac agcatactgc ttggaatgtg tgaggatgtt 480

atttacgctc gagtttctgt tcggactgtg ctggatgctt gcagtgccca cattaggaat 540

agcaattgtg caccctcatt ttcctacgtg aaacacttgg taaaactggt tctgggaaat 600

ctttctggga cagatcagct ttcctgtaac agtgaacaaa agcctgatcg aagccaggct 660

attcgagatc gattgcgagg aaaaggatta ccaacaggaa gaagctctac ttctgatgta 720

ctagacatac aaaagcctcc actctctcat cagacctttc ttaacaaagg gcttagtaaa 780

tctatgggat ttctgtccat caaagataca caagatgaga attatttcaa ggacatttta 840

tcagataatt ctggacgtga agattctgaa aatacattct ccccttacca gttcaaaact 900

agtggcccag aaaaaaaacc catccctggc attgatgtgc tttctaagaa gaagatctgg 960

gcttcatcca tggacttgct ttgtacagct gacagagact tctcttcagg agagactgcc 1020

acatatcgtc gttgtcaccc tgaggcagta acagtgcgga cttcaactac tcctagaaaa 1080

aaggaggcaa gatactcaga tggaagtata gccttggata tctttggccc tcagaaaatg 1140

gatccaatat atcacactcg agaattgccc acctcctcag caatatcaag tgctttggac 1200

cgaatccgag agagacaaaa gaaacttcag gttctgaggg aagccatgaa tgtagaagaa 1260

ccagttcgaa gatacaaaac ttatcatggt gatgtcttta gtacctccag tgaaagtcca 1320

tctattattt cctctgaatc agatttcaga caagtgagaa gaagtgaagc ctcaaagagg 1380

tttgaatcca gcagtggtct cccaggggta gatgaaacct taagtcaagg ccagtcacag 1440

agaccgagca gacaatatga aacacccttt gaaggcaact taattaatca agagatcatg 1500
ctaaaacggc aagaggaaga actgatgcag ctacaagcca aaatggccct tagacagtct 1560
cggttgagcc tatatccagg agacacaatc aaagcgtcca tgcttgacat caccagggat 1620
ccgttaagag aaattgccct agaaacagcc atgactcaaa gaaaactgag gaatttcttt 1680
ggccctgagt ttgtgaaaat gacaattgaa ccatttatat ctttggattt gccacggtct 1740
attcttacta agaaagggaa gaatgaggat aaccgaagga aagtaaacat aatgcttctg 1800
aacgggcaaa gactggaact gacctgtgat accaaaacta tatgtaaaga tgtgtttgat 1860
atggttgtgg cacatattgg cttagtagag catcatttgt ttgctttagc taccctcaaa 1920
gataatgaat atttctttgt tgatcctgac ttaaaattaa ccaaagtggc cccagaggga 1980
tggaaagaag aaccaaagaa aaagaccaaa gccactgtta attttacttt gtttttcaga 2040
attaaatttt ttatggatga tgttagtcta atacaacata ctctgacgtg tcatcagtat 2100
taccttcagc ttcgaaaaga tattttggag gaaaggatgc actgtgatga tgagacttcc 2160
ttattgctgg catccttggc tctccaggct gagtatggag attatcaacc agaggttcat 2220
ggtgtgtctt actttagaat ggagcactat ttgcccgcca gagtgatgga gaaacttgat 2280
ttatcctata tcaaagaaga gttacccaaa ttgcataata cctatgtggg agcttctgaa 2340
aaagagacag agttagaatt tttaaaggtc tgccaaagac tgacagaata tggagttcat 2400
tttcaccgag tgcaccctga gaagaagtca caaacaggaa tattgcttgg agtctgttct 2460
aaaggtgtcc ttgtgtttga agttcacaat ggagtgcgca cattggtcct tcgctttcca 2520
tggagggaaa ccaagaaaat atctttttct aaaaagaaaa tcacattgca aaatacatca 2580
gatggaataa aacatggctt ccagacagac aacagtaaga tatgccagta cctgctgcac 2640
ctctgctctt accagcataa gttccagcta cagatgagag caagacagag caaccaagat 2700
gcccaagata ttgagagagc ttcgtttagg agcctgaatc tccaagcaga gtctgttaga 2760
ggatttaata tgggacgagc aatcagcact ggcagtctgg ccagcagcac cctcaacaaa 2820
cttgctgttc gacctttatc agttcaagct gagattctga agaggctatc ctgctcagag 2880
ctgtcgcttt accagccatt gcaaaacagt tcaaaagaga agaatgacaa agcttcatgg 2940
gaggaaaagc ctagagagat gagtaaatca taccatgatc tcagtcaggc ctctctctat 3000
ccacatcgga aaaatgtcat tgttaacatg gaacccccac cacaaaccgt tgcagagttg 3060
gtgggaaaac cttctcacca gatgtcaaga tctgatgcag aatctttggc aggagtgaca 3120
aaacttaata attcaaagtc tgttgcgagt ttaaatagaa gtcctgaaag gaggaaacat 3180
gaatcagact cctcatccat tgaagaccct gggcaagcat atgttctaga tgtgctacac 3240
aaaagatgga gcatagtatc ttcaccagaa agggagatca ccttagtgaa cctgaaaaaa 3300
gatgcaaagt atggcttggg atttcaaatt attggtgggg agaagatggg aagactggac 3360
ctaggcatat ttatcagttc agttgcccct ggaggaccag ctgacttgga tggatgcttg 3420
aagccaggag accgtttgat atctgtgaat agtgtgagtc tggagggagt cagccaccat 3480

gctgcaattg aaattttgca aaatgcacct gaagatgtga cacttgttat ctctcagcca 3540
aaagaaaaga tatccaaagt gccttctact cctgtgcatc tcaccaatga gatgaaaaac 3600
tacatgaaga aatcttccta catgcaagac agtgctatag attcttcttc caaggatcac 3660
cactggtcac gtggtaccct gaggcacatc tcggagaact cctttgggcc atctgggggc 3720
ctgcgggaag gaagcctgag ttctcaagat tccaggactg agagtgccag cttgtctcaa 3780
agccaggtca atggtttctt tgccagccat ttaggtgacc aaacctggca ggaatcacag 3840
catggcagcc cttccccatc tgtaatatcc aaagccaccg agaaagagac tttcactgat 3900
agtaaccaaa gcaaaactaa aaagccaggc atttctgatg taactgatta ctcagaccgt 3960
ggagattcag acatggatga agccacttac tccagcagtc aggatcatca aacaccaaaa 4020
caggaatctt cctcttcagt gaatacatcc aacaagatga attttaaaac tttttcttca 4080
tcacctccta agcctggaga tatctttgag gttgaactgg ctaaaaatga taacagcttg 4140
gggataagtg tcacgggagg tgtgaatacg agtgtcagac atggtggcat ttatgtgaaa 4200
gctgttattc cccagggagc agcagagtct gatggtagaa ttcacaaagg tgatcgcgtc 4260
ctagctgtca atggagttag tctagaagga gccacccata agcaagctgt ggaaacactg 4320
agaaatacag gacaggtggt tcatctgtta ttagaaaagg gacaatctcc aacatctaaa 4380
gaacatgtcc cggtaacccc acagtgtacc ctttcagatc agaatgccca aggtcaaggc 4440
ccagaaaaag tgaagaaaac aactcaggtc aaagactaca gctttgtcac tgaagaaaat 4500
acatttgagg taaaattatt taaaaatagc tcaggtctag gattcagttt ttctcgagaa 4560
gataatctta taccggagca aattaatgcc agcatagtaa gggttaaaaa gctctttcct 4620
ggacagccag cagcagaaag tggaaaaatt gatgtaggag atgttatctt gaaagtgaat 4680
ggagcctctt tgaaaggact atctcagcag gaagtcatat ctgctctcag gggaactgct 4740
ccagaagtat tcttgcttct ctgcagacct ccacctggtg tgctaccgga aattgatact 4800
gcgcttttga ccccacttca gtctccagca caagtacttc caaacagcag taaagactct 4860
tctcagccat catgtgtgga gcaaagcacc agctcagatg aaaatgaaat gtcagacaaa 4920
agcaaaaaac agtgcaagtc cccatccaga agagacagtt acagtgacag cagtgggagt 4980
ggagaagatg acttagtgac agctccagca aacatatcaa attcgacctg gagttcagct 5040
ttgcatcaga ctctaagcaa catggtatca caggcacaga gtcatcatga agcacccaag 5100
agtcaagaag ataccatttg taccatgttt tactatcctc agaaaattcc caataaacca 5160
gagtttgagg acagtaatcc ttcccctcta ccaccggata tggctcctgg gcagagttat 5220
caaccccaat cagaatctgc ttcctctagt tcgatggata agtatcatat acatcacatt 5280
tctgaaccaa ctagacaaga aaactggaca cctttgaaaa atgacttgga aaatcacctt 5340
gaagactttg aactggaagt agaactcctc attaccctaa ttaaatcaga aaaaggaagc 5400
ctgggtttta cagtaaccaa aggcaatcag agaattggtt gttatgttca tgatgtcata 5460
caggatccag ccaaaagtga tggaaggcta aaacctgggg accggctcat aaaggttaat 5520

gatacagatg ttactaatat gactcataca gatgcagtta atctgctccg ggctgcatcc 5580
aaaacagtca gattagttat tggacgagtt ctagaattac ccagaatacc aatgttgcct 5640
catttgctac cggacataac actaacgtgc aacaaagagg agttgggttt ttccttatgt 5700
ggaggtcatg acagccttta tcaagtggta tatattagtg atattaatcc aaggtccgtc 5760
gcagccattg agggtaatct ccagctatta gatgtcatcc attatgtgaa cggagtcagc 5820
acacaaggaa tgaccttgga ggaagttaac agagcattag acatgtcact tccttcattg 5880
gtattgaaag caacaagaaa tgatcttcca gtggtcccca gctcaaagag gtctgctgtt 5940
tcagctccaa agtcaaccaa aggcaatggt tcctacagtg tggggtcttg cagccagcct 6000
gccctcactc ctaatgattc attctccacg gttgctgggg aagaaataaa tgaaatatcg 6060
taccccaaag gaaaatgttc tacttatcag ataaagggat caccaaactt gactctgccc 6120
aaagaatctt atatacaaga agatgacatt tatgatgatt cccaagaagc tgaagttatc 6180
cagtctctgc tggatgttgt ggatgaggaa gcccagaatc ttttaaacga aaataatgca 6240
gcaggatact cctgtggtcc aggtacatta aagatgaatg ggaagttatc agaagagaga 6300
acagaagata cagactgcga tggttcacct ttacctgagt attttactga ggccaccaaa 6360
atgaatggct gtgaagaata ttgtgaagaa aaagtaaaaa gtgaaagctt aattcagaag 6420
ccacaagaaa agaagactga tgatgatgaa ataacatggg gaaatgatga gttgccaata 6480
gagagaacaa accatgaaga ttctgataaa gatcattcct ttctgacaaa cgatgagctc 6540
gctgtactcc ctgtcgtcaa agtgcttccc tctggtaaat acacgggtgc caacttaaaa 6600
tcagtcattc gagtcctgcg gggtttgcta gatcaaggaa ttccttctaa ggagctggag 6660
aatcttcaag aattaaaacc tttggatcag tgtctaattg ggcaaactaa ggaaaacaga 6720
aggaagaaca gatataaaaa tatacttccc tatgatgcta caagagtgcc tcttggagat 6780
gaaggtggct atatcaatgc cagcttcatt aagataccag ttgggaaaga agagttcgtt 6840
tacattgcct gccaaggacc actgcctaca actgttggag acttctggca gatgatttgg 6900
gagcaaaaat ccacagtgat agccatgatg actcaagaag tagaaggaga aaaaatcaaa 6960
tgccagcgct attggcccaa catcctaggc aaaacaacaa tggtcagcaa cagacttcga 7020
ctggctcttg tgagaatgca gcagctgaag ggctttgtgg tgagggcaat gacccttgaa 7080
gatattcaga ccagagaggt gcgccatatt tctcatctga atttcactgc ctggccagac 7140
catgatacac cttctcaacc agatgatctg cttactttta tctcctacat gagacacatc 7200
cacagatcag gcccaatcat tacgcactgc agtgctggca ttggacgttc agggaccctg 7260
atttgcatag atgtggttct gggattaatc agtcaggatc ttgattttga catctctgat 7320
ttggtgcgct gcatgagact acaaagacac ggaatggttc agacagagga tcaatatatt 7380
ttctgctatc aagtcatcct ttatgtcctg acacgtcttc aagcagaaga agagcaaaaa 7440
cagcagcctc agcttctgaa gtgacatgaa aagagcctct ggatgcattt ccatttctct 7500
ccttaacctc cagcagactc ctgctctcta tccaaaataa agatcacaga gcagcaagtt 7560

catacaacat gcatgttctc ctctatctta gaggggtatt cttcttgaaa ataaaaaata 7620

ttgaaatgct gtatttttac agctacttta acctatgata attatttaca aaattttaac 7680

actaaccaaa caatgcagat cttagggatg attaaaggca gcatttgatg atagcagaca 7740

ttgttacaag gacatggtga gtctattttt aatgcaccaa tcttgtttat agcaaaaatg 7800

ttttccaata ttttaataaa gtagttattt tataggggat acttgaaacc agtatttaag 7860

ctttaaatga cagtaatatt ggcatagaaa aaagtagcaa atgtttactg tatcaatttc 7920

taatgtttac tatatagaat ttcctgtaat atatttatat actttttcat gaaaatggag 7980

ttatcagtta tctgtttgtt actgcatcat ctgtttgtaa tcattatctc actttgtaaa 8040

taaaaacaca ccttaaaaca tg 8062

<210> 5
<211> 2485
<212> PRT
<213> Homo sapiens

<400> 5

Met His Val Ser Leu Ala Glu Ala Leu Glu Val Arg Gly Gly Pro Leu
1               5                   10                  15

Gln Glu Glu Glu Ile Trp Ala Val Leu Asn Gln Ser Ala Glu Ser Leu
            20                  25                  30

Gln Glu Leu Phe Arg Lys Val Ser Leu Ala Asp Pro Ala Ala Leu Gly
        35                  40                  45

Phe Ile Ile Ser Pro Trp Ser Leu Leu Leu Leu Pro Ser Gly Ser Val
    50                  55                  60

Ser Phe Thr Asp Glu Asn Ile Ser Asn Gln Asp Leu Arg Ala Phe Thr
65                  70                  75                  80

Ala Pro Glu Val Leu Gln Asn Gln Ser Leu Thr Ser Leu Ser Asp Val
                85                  90                  95

Glu Lys Ile His Ile Tyr Ser Leu Gly Met Thr Leu Tyr Trp Gly Ala
            100                 105                 110

Asp Tyr Glu Val Pro Gln Ser Gln Pro Ile Lys Leu Gly Asp His Leu
        115                 120                 125

Asn Ser Ile Leu Leu Gly Met Cys Glu Asp Val Ile Tyr Ala Arg Val
    130                 135                 140

Ser Val Arg Thr Val Leu Asp Ala Cys Ser Ala His Ile Arg Asn Ser
145                 150                 155                 160

Asn Cys Ala Pro Ser Phe Ser Tyr Val Lys His Leu Val Lys Leu Val
                165                 170                 175

Leu Gly Asn Leu Ser Gly Thr Asp Gln Leu Ser Cys Asn Ser Glu Gln
            180                 185                 190

Lys Pro Asp Arg Ser Gln Ala Ile Arg Asp Arg Leu Arg Gly Lys Gly
        195                 200                 205

Leu Pro Thr Gly Arg Ser Ser Thr Ser Asp Val Leu Asp Ile Gln Lys
    210                 215                 220

Pro Pro Leu Ser His Gln Thr Phe Leu Asn Lys Gly Leu Ser Lys Ser
225                 230                 235                 240

Met Gly Phe Leu Ser Ile Lys Asp Thr Gln Asp Glu Asn Tyr Phe Lys
                245                 250                 255

Asp Ile Leu Ser Asp Asn Ser Gly Arg Glu Asp Ser Glu Asn Thr Phe
            260                 265                 270

Ser Pro Tyr Gln Phe Lys Thr Ser Gly Pro Glu Lys Lys Pro Ile Pro
        275                 280                 285

Gly Ile Asp Val Leu Ser Lys Lys Lys Ile Trp Ala Ser Ser Met Asp
    290                 295                 300

Leu Leu Cys Thr Ala Asp Arg Asp Phe Ser Ser Gly Glu Thr Ala Thr
305                 310                 315                 320

Tyr Arg Arg Cys His Pro Glu Ala Val Thr Val Arg Thr Ser Thr Thr
                325                 330                 335

Pro Arg Lys Lys Glu Ala Arg Tyr Ser Asp Gly Ser Ile Ala Leu Asp
            340                 345                 350

Ile Phe Gly Pro Gln Lys Met Asp Pro Ile Tyr His Thr Arg Glu Leu
        355                 360                 365

Pro Thr Ser Ser Ala Ile Ser Ser Ala Leu Asp Arg Ile Arg Glu Arg
    370                 375                 380

Gln Lys Lys Leu Gln Val Leu Arg Glu Ala Met Asn Val Glu Glu Pro
385                 390                 395                 400

Val Arg Arg Tyr Lys Thr Tyr His Gly Asp Val Phe Ser Thr Ser Ser
                405                 410                 415

Glu Ser Pro Ser Ile Ile Ser Ser Glu Ser Asp Phe Arg Gln Val Arg
            420                 425                 430

Arg Ser Glu Ala Ser Lys Arg Phe Glu Ser Ser Ser Gly Leu Pro Gly
        435                 440                 445

Val Asp Glu Thr Leu Ser Gln Gly Gln Ser Gln Arg Pro Ser Arg Gln
    450                 455                 460

Tyr Glu Thr Pro Phe Glu Gly Asn Leu Ile Asn Gln Glu Ile Met Leu
465                 470                 475                 480

Lys Arg Gln Glu Glu Glu Leu Met Gln Leu Gln Ala Lys Met Ala Leu
                485                 490                 495

Arg Gln Ser Arg Leu Ser Leu Tyr Pro Gly Asp Thr Ile Lys Ala Ser
            500                 505                 510

Met Leu Asp Ile Thr Arg Asp Pro Leu Arg Glu Ile Ala Leu Glu Thr
        515                 520                 525

Ala Met Thr Gln Arg Lys Leu Arg Asn Phe Phe Gly Pro Glu Phe Val
    530                 535                 540

Lys Met Thr Ile Glu Pro Phe Ile Ser Leu Asp Leu Pro Arg Ser Ile
545                 550                 555                 560

Leu Thr Lys Lys Gly Lys Asn Glu Asp Asn Arg Arg Lys Val Asn Ile
                565                 570                 575

Met Leu Leu Asn Gly Gln Arg Leu Glu Leu Thr Cys Asp Thr Lys Thr
            580                 585                 590

Ile Cys Lys Asp Val Phe Asp Met Val Val Ala His Ile Gly Leu Val
        595                 600                 605

Glu His His Leu Phe Ala Leu Ala Thr Leu Lys Asp Asn Glu Tyr Phe
    610                 615                 620

Phe Val Asp Pro Asp Leu Lys Leu Thr Lys Val Ala Pro Glu Gly Trp
625                 630                 635                 640

Lys Glu Glu Pro Lys Lys Lys Thr Lys Ala Thr Val Asn Phe Thr Leu
                645                 650                 655

Phe Phe Arg Ile Lys Phe Phe Met Asp Asp Val Ser Leu Ile Gln His
            660                 665                 670

Thr Leu Thr Cys His Gln Tyr Tyr Leu Gln Leu Arg Lys Asp Ile Leu
        675                 680                 685

Glu Glu Arg Met His Cys Asp Asp Glu Thr Ser Leu Leu Leu Ala Ser
    690                 695                 700

Leu Ala Leu Gln Ala Glu Tyr Gly Asp Tyr Gln Pro Glu Val His Gly
705                 710                 715                 720

Val Ser Tyr Phe Arg Met Glu His Tyr Leu Pro Ala Arg Val Met Glu
                725                 730                 735

Lys Leu Asp Leu Ser Tyr Ile Lys Glu Glu Leu Pro Lys Leu His Asn
            740                 745                 750

Thr Tyr Val Gly Ala Ser Glu Lys Glu Thr Glu Leu Glu Phe Leu Lys
        755                 760                 765

Val Cys Gln Arg Leu Thr Glu Tyr Gly Val His Phe His Arg Val His
    770                 775                 780

Pro Glu Lys Lys Ser Gln Thr Gly Ile Leu Leu Gly Val Cys Ser Lys
785                 790                 795                 800

Gly Val Leu Val Phe Glu Val His Asn Gly Val Arg Thr Leu Val Leu
                805                 810                 815

Arg Phe Pro Trp Arg Glu Thr Lys Lys Ile Ser Phe Ser Lys Lys Lys
            820                 825                 830

Ile Thr Leu Gln Asn Thr Ser Asp Gly Ile Lys His Gly Phe Gln Thr
        835                 840                 845

Asp Asn Ser Lys Ile Cys Gln Tyr Leu Leu His Leu Cys Ser Tyr Gln
    850                 855                 860

His Lys Phe Gln Leu Gln Met Arg Ala Arg Gln Ser Asn Gln Asp Ala
865                 870                 875                 880

Gln Asp Ile Glu Arg Ala Ser Phe Arg Ser Leu Asn Leu Gln Ala Glu
                885                 890                 895

Ser Val Arg Gly Phe Asn Met Gly Arg Ala Ile Ser Thr Gly Ser Leu
            900                 905                 910

Ala Ser Ser Thr Leu Asn Lys Leu Ala Val Arg Pro Leu Ser Val Gln
        915                 920                 925

Ala Glu Ile Leu Lys Arg Leu Ser Cys Ser Glu Leu Ser Leu Tyr Gln
    930                 935                 940

Pro Leu Gln Asn Ser Ser Lys Glu Lys Asn Asp Lys Ala Ser Trp Glu
945                 950                 955                 960

Glu Lys Pro Arg Glu Met Ser Lys Ser Tyr His Asp Leu Ser Gln Ala
                965                 970                 975

Ser Leu Tyr Pro His Arg Lys Asn Val Ile Val Asn Met Glu Pro Pro
            980                 985                 990

Pro Gln Thr Val Ala Glu Leu Val Gly Lys Pro Ser His Gln Met Ser
        995                 1000                1005

Arg Ser Asp Ala Glu Ser Leu Ala Gly Val Thr Lys Leu Asn Asn
    1010                1015                1020

Ser Lys Ser Val Ala Ser Leu Asn Arg Ser Pro Glu Arg Arg Lys
    1025                1030                1035

His Glu Ser Asp Ser Ser Ser Ile Glu Asp Pro Gly Gln Ala Tyr
    1040                1045                1050

Val Leu Gly Met Thr Met His Ser Ser Gly Asn Ser Ser Ser Gln
    1055                1060                1065

Val Pro Leu Lys Glu Asn Asp Val Leu His Lys Arg Trp Ser Ile
    1070                1075                1080

Val Ser Ser Pro Glu Arg Glu Ile Thr Leu Val Asn Leu Lys Lys
    1085                1090                1095

Asp Ala Lys Tyr Gly Leu Gly Phe Gln Ile Ile Gly Gly Glu Lys
    1100                1105                1110

Met Gly Arg Leu Asp Leu Gly Ile Phe Ile Ser Ser Val Ala Pro
    1115                1120                1125

Gly Gly Pro Ala Asp Leu Asp Gly Cys Leu Lys Pro Gly Asp Arg
    1130                1135                1140

Leu Ile Ser Val Asn Ser Val Ser Leu Glu Gly Val Ser His His
    1145                1150                1155

Ala Ala Ile Glu Ile Leu Gln Asn Ala Pro Glu Asp Val Thr Leu
    1160                1165                1170

Val Ile Ser Gln Pro Lys Glu Lys Ile Ser Lys Val Pro Ser Thr
    1175                1180                1185

Pro Val His Leu Thr Asn Glu Met Lys Asn Tyr Met Lys Lys Ser
    1190                1195                1200

Ser Tyr Met Gln Asp Ser Ala Ile Asp Ser Ser Ser Lys Asp His
    1205                1210                1215

His Trp Ser Arg Gly Thr Leu Arg His Ile Ser Glu Asn Ser Phe
    1220                1225                1230

Gly Pro Ser Gly Gly Leu Arg Glu Gly Ser Leu Ser Ser Gln Asp
    1235                1240                1245

Ser Arg Thr Glu Ser Ala Ser Leu Ser Gln Ser Gln Val Asn Gly
    1250                1255                1260

Phe Phe Ala Ser His Leu Gly Asp Gln Thr Trp Gln Glu Ser Gln
    1265                1270                1275

His Gly Ser Pro Ser Pro Ser Val Ile Ser Lys Ala Thr Glu Lys
    1280                1285                1290

Glu Thr Phe Thr Asp Ser Asn Gln Ser Lys Thr Lys Lys Pro Gly
    1295                1300                1305

Ile Ser Asp Val Thr Asp Tyr Ser Asp Arg Gly Asp Ser Asp Met
    1310                1315                1320

Asp Glu Ala Thr Tyr Ser Ser Ser Gln Asp His Gln Thr Pro Lys
    1325                1330                1335

Gln Glu Ser Ser Ser Ser Val Asn Thr Ser Asn Lys Met Asn Phe
    1340                1345                1350

Lys Thr Phe Ser Ser Ser Pro Pro Lys Pro Gly Asp Ile Phe Glu
    1355                1360                1365

Val Glu Leu Ala Lys Asn Asp Asn Ser Leu Gly Ile Ser Val Thr
    1370                1375                1380

Gly Gly Val Asn Thr Ser Val Arg His Gly Gly Ile Tyr Val Lys
    1385                1390                1395

Ala Val Ile Pro Gln Gly Ala Ala Glu Ser Asp Gly Arg Ile His
    1400                1405                1410

Lys Gly Asp Arg Val Leu Ala Val Asn Gly Val Ser Leu Glu Gly
    1415                1420                1425

Ala Thr His Lys Gln Ala Val Glu Thr Leu Arg Asn Thr Gly Gln
    1430                1435                1440

Val Val His Leu Leu Leu Glu Lys Gly Gln Ser Pro Thr Ser Lys
    1445                1450                1455

Glu His Val Pro Val Thr Pro Gln Cys Thr Leu Ser Asp Gln Asn
    1460                1465                1470

Ala Gln Gly Gln Gly Pro Glu Lys Val Lys Lys Thr Thr Gln Val
    1475                1480                1485

Lys Asp Tyr Ser Phe Val Thr Glu Glu Asn Thr Phe Glu Val Lys
    1490                1495                1500

Leu Phe Lys Asn Ser Ser Gly Leu Gly Phe Ser Phe Ser Arg Glu
    1505                1510                1515

Asp Asn Leu Ile Pro Glu Gln Ile Asn Ala Ser Ile Val Arg Val
    1520                1525                1530

Lys Lys Leu Phe Pro Gly Gln Pro Ala Ala Glu Ser Gly Lys Ile
    1535                1540                1545

Asp Val Gly Asp Val Ile Leu Lys Val Asn Gly Ala Ser Leu Lys
    1550                1555                1560

Gly Leu Ser Gln Gln Glu Val Ile Ser Ala Leu Arg Gly Thr Ala
    1565                1570                1575

Pro Glu Val Phe Leu Leu Leu Cys Arg Pro Pro Pro Gly Val Leu
    1580                1585                1590

Pro Glu Ile Asp Thr Ala Leu Leu Thr Pro Leu Gln Ser Pro Ala
    1595                1600                1605

Gln Val Leu Pro Asn Ser Ser Lys Asp Ser Ser Gln Pro Ser Cys
    1610                1615                1620

Val Glu Gln Ser Thr Ser Ser Asp Glu Asn Glu Met Ser Asp Lys
    1625                1630                1635

Ser Lys Lys Gln Cys Lys Ser Pro Ser Arg Arg Asp Ser Tyr Ser
    1640                1645                1650

Asp Ser Ser Gly Ser Gly Glu Asp Asp Leu Val Thr Ala Pro Ala
    1655                1660                1665

Asn Ile Ser Asn Ser Thr Trp Ser Ser Ala Leu His Gln Thr Leu
    1670                1675                1680

Ser Asn Met Val Ser Gln Ala Gln Ser His His Glu Ala Pro Lys
    1685                1690                1695

Ser Gln Glu Asp Thr Ile Cys Thr Met Phe Tyr Tyr Pro Gln Lys
    1700                1705                1710

Ile Pro Asn Lys Pro Glu Phe Glu Asp Ser Asn Pro Ser Pro Leu
    1715                1720                1725

Pro Pro Asp Met Ala Pro Gly Gln Ser Tyr Gln Pro Gln Ser Glu
    1730                1735                1740

Ser Ala Ser Ser Ser Ser Met Asp Lys Tyr His Ile His His Ile
    1745                1750                1755

Ser Glu Pro Thr Arg Gln Glu Asn Trp Thr Pro Leu Lys Asn Asp
    1760                1765                1770

Leu Glu Asn His Leu Glu Asp Phe Glu Leu Glu Val Glu Leu Leu
    1775                1780                1785

Ile Thr Leu Ile Lys Ser Glu Lys Gly Ser Leu Gly Phe Thr Val
    1790                1795                1800

Thr Lys Gly Asn Gln Arg Ile Gly Cys Tyr Val His Asp Val Ile
    1805                1810                1815

Gln Asp Pro Ala Lys Ser Asp Gly Arg Leu Lys Pro Gly Asp Arg
    1820                1825                1830

Leu Ile Lys Val Asn Asp Thr Asp Val Thr Asn Met Thr His Thr
    1835                1840                1845

Asp Ala Val Asn Leu Leu Arg Ala Ala Ser Lys Thr Val Arg Leu
    1850                1855                1860

Val Ile Gly Arg Val Leu Glu Leu Pro Arg Ile Pro Met Leu Pro
    1865                1870                1875

His Leu Leu Pro Asp Ile Thr Leu Thr Cys Asn Lys Glu Glu Leu
    1880                1885                1890

Gly Phe Ser Leu Cys Gly Gly His Asp Ser Leu Tyr Gln Val Val
    1895                1900                1905

Tyr Ile Ser Asp Ile Asn Pro Arg Ser Val Ala Ala Ile Glu Gly
    1910                1915                1920

Asn Leu Gln Leu Leu Asp Val Ile His Tyr Val Asn Gly Val Ser
    1925                1930                1935

Thr Gln Gly Met Thr Leu Glu Glu Val Asn Arg Ala Leu Asp Met
    1940                1945                1950

Ser Leu Pro Ser Leu Val Leu Lys Ala Thr Arg Asn Asp Leu Pro
    1955                1960                1965

Val Val Pro Ser Ser Lys Arg Ser Ala Val Ser Ala Pro Lys Ser
    1970                1975                1980

Thr Lys Gly Asn Gly Ser Tyr Ser Val Gly Ser Cys Ser Gln Pro
    1985                1990                1995

Ala Leu Thr Pro Asn Asp Ser Phe Ser Thr Val Ala Gly Glu Glu
    2000                2005                2010

Ile Asn Glu Ile Ser Tyr Pro Lys Gly Lys Cys Ser Thr Tyr Gln
    2015                2020                2025

Ile Lys Gly Ser Pro Asn Leu Thr Leu Pro Lys Glu Ser Tyr Ile
    2030                2035                2040

Gln Glu Asp Asp Ile Tyr Asp Asp Ser Gln Glu Ala Glu Val Ile
    2045                2050                2055

Gln Ser Leu Leu Asp Val Val Asp Glu Glu Ala Gln Asn Leu Leu
    2060                2065                2070

Asn Glu Asn Asn Ala Ala Gly Tyr Ser Cys Gly Pro Gly Thr Leu
    2075                2080                2085

Lys Met Asn Gly Lys Leu Ser Glu Glu Arg Thr Glu Asp Thr Asp
    2090                2095                2100

Cys Asp Gly Ser Pro Leu Pro Glu Tyr Phe Thr Glu Ala Thr Lys
    2105                2110                2115

Met Asn Gly Cys Glu Glu Tyr Cys Glu Glu Lys Val Lys Ser Glu
    2120                2125                2130

Ser Leu Ile Gln Lys Pro Gln Glu Lys Lys Thr Asp Asp Asp Glu
    2135                2140                2145

Ile Thr Trp Gly Asn Asp Glu Leu Pro Ile Glu Arg Thr Asn His
    2150                2155                2160

Glu Asp Ser Asp Lys Asp His Ser Phe Leu Thr Asn Asp Glu Leu
    2165                2170                2175

Ala Val Leu Pro Val Val Lys Val Leu Pro Ser Gly Lys Tyr Thr
    2180                2185                2190

Gly Ala Asn Leu Lys Ser Val Ile Arg Val Leu Arg Gly Leu Leu
    2195                2200                2205

Asp Gln Gly Ile Pro Ser Lys Glu Leu Glu Asn Leu Gln Glu Leu
    2210                2215                2220

Lys Pro Leu Asp Gln Cys Leu Ile Gly Gln Thr Lys Glu Asn Arg
    2225                2230                2235

Arg Lys Asn Arg Tyr Lys Asn Ile Leu Pro Tyr Asp Ala Thr Arg
    2240                2245                2250

Val Pro Leu Gly Asp Glu Gly Gly Tyr Ile Asn Ala Ser Phe Ile
    2255                2260                2265

Lys Ile Pro Val Gly Lys Glu Glu Phe Val Tyr Ile Ala Cys Gln
    2270                2275                2280

Gly Pro Leu Pro Thr Thr Val Gly Asp Phe Trp Gln Met Ile Trp
    2285                2290                2295

Glu Gln Lys Ser Thr Val Ile Ala Met Met Thr Gln Glu Val Glu
    2300                2305                2310

Gly Glu Lys Ile Lys Cys Gln Arg Tyr Trp Pro Asn Ile Leu Gly
    2315                2320                2325

Lys Thr Thr Met Val Ser Asn Arg Leu Arg Leu Ala Leu Val Arg
    2330                2335                2340

Met Gln Gln Leu Lys Gly Phe Val Val Arg Ala Met Thr Leu Glu
    2345                2350                2355

Asp Ile Gln Thr Arg Glu Val Arg His Ile Ser His Leu Asn Phe
    2360                2365                2370

Thr Ala Trp Pro Asp His Asp Thr Pro Ser Gln Pro Asp Asp Leu
    2375                2380                2385

Leu Thr Phe Ile Ser Tyr Met Arg His Ile His Arg Ser Gly Pro
    2390                2395                2400

Ile Ile Thr His Cys Ser Ala Gly Ile Gly Arg Ser Gly Thr Leu
    2405                2410                2415

Ile Cys Ile Asp Val Val Leu Gly Leu Ile Ser Gln Asp Leu Asp
    2420                2425                2430

Phe Asp Ile Ser Asp Leu Val Arg Cys Met Arg Leu Gln Arg His
    2435                2440                2445

Gly Met Val Gln Thr Glu Asp Gln Tyr Ile Phe Cys Tyr Gln Val
    2450                2455                2460

Ile Leu Tyr Val Leu Thr Arg Leu Gln Ala Glu Glu Glu Gln Lys
    2465                2470                2475

Gln Gln Pro Gln Leu Leu Lys
    2480                2485

<210>  6
<211>  2294
<212>  PRT
<213>  Homo sapiens

<400>  6

Met His Val Ser Leu Ala Glu Ala Leu Glu Val Arg Gly Gly Pro Leu
1               5                   10                  15

Gln Glu Glu Glu Ile Trp Ala Val Leu Asn Gln Ser Ala Glu Ser Leu
            20                  25                  30

Gln Glu Leu Phe Arg Lys Val Ser Leu Ala Asp Pro Ala Ala Leu Gly
        35                  40                  45

Phe Ile Ile Ser Pro Trp Ser Leu Leu Leu Leu Pro Ser Gly Ser Val
    50                  55                  60

Ser Phe Thr Asp Glu Asn Ile Ser Asn Gln Asp Leu Arg Ala Phe Thr
65                  70                  75                  80

Ala Pro Glu Val Leu Gln Asn Gln Ser Leu Thr Ser Leu Ser Asp Val
                85                  90                  95

Glu Lys Ile His Ile Tyr Ser Leu Gly Met Thr Leu Tyr Trp Gly Ala
            100                 105                 110

Asp Tyr Glu Val Pro Gln Ser Gln Pro Ile Lys Leu Gly Asp His Leu
        115                 120                 125

Asn Ser Ile Leu Leu Gly Met Cys Glu Asp Val Ile Tyr Ala Arg Val
    130                 135                 140

Ser Val Arg Thr Val Leu Asp Ala Cys Ser Ala His Ile Arg Asn Ser
145                 150                 155                 160

Asn Cys Ala Pro Ser Phe Ser Tyr Val Lys His Leu Val Lys Leu Val
                165                 170                 175

Leu Gly Asn Leu Ser Gly Thr Asp Gln Leu Ser Cys Asn Ser Glu Gln
            180                 185                 190

Lys Pro Asp Arg Ser Gln Ala Ile Arg Asp Arg Leu Arg Gly Lys Gly
        195                 200                 205

Leu Pro Thr Gly Arg Ser Ser Thr Ser Asp Val Leu Asp Ile Gln Lys
    210                 215                 220

Pro Pro Leu Ser His Gln Thr Phe Leu Asn Lys Gly Leu Ser Lys Ser
225                 230                 235                 240

Met Gly Phe Leu Ser Ile Lys Asp Thr Gln Asp Glu Asn Tyr Phe Lys
                245                 250                 255

Asp Ile Leu Ser Asp Asn Ser Gly Arg Glu Asp Ser Glu Asn Thr Phe
            260                 265                 270

Ser Pro Tyr Gln Phe Lys Thr Ser Gly Pro Glu Lys Lys Pro Ile Pro
        275                 280                 285

Gly Ile Asp Val Leu Ser Lys Lys Lys Ile Trp Ala Ser Ser Met Asp
    290                 295                 300

Leu Leu Cys Thr Ala Asp Arg Asp Phe Ser Ser Gly Glu Thr Ala Thr
305                 310                 315                 320

Tyr Arg Arg Cys His Pro Glu Ala Val Thr Val Arg Thr Ser Thr Thr
                325                 330                 335

Pro Arg Lys Lys Glu Ala Arg Tyr Ser Asp Gly Ser Ile Ala Leu Asp
            340                 345                 350

Ile Phe Gly Pro Gln Lys Met Asp Pro Ile Tyr His Thr Arg Glu Leu
        355                 360                 365

Pro Thr Ser Ser Ala Ile Ser Ser Ala Leu Asp Arg Ile Arg Glu Arg
    370                 375                 380

Gln Lys Lys Leu Gln Val Leu Arg Glu Ala Met Asn Val Glu Glu Pro
385                 390                 395                 400

Val Arg Arg Tyr Lys Thr Tyr His Gly Asp Val Phe Ser Thr Ser Ser
                405                 410                 415

Glu Ser Pro Ser Ile Ile Ser Ser Glu Ser Asp Phe Arg Gln Val Arg
            420                 425                 430

Arg Ser Glu Ala Ser Lys Arg Phe Glu Ser Ser Ser Gly Leu Pro Gly
        435                 440                 445

Val Asp Glu Thr Leu Ser Gln Gly Gln Ser Gln Arg Pro Ser Arg Gln
    450                 455                 460

Tyr Glu Thr Pro Phe Glu Gly Asn Leu Ile Asn Gln Glu Ile Met Leu
465                 470                 475                 480

Lys Arg Gln Glu Glu Glu Leu Met Gln Leu Gln Ala Lys Met Ala Leu
                485                 490                 495

Arg Gln Ser Arg Leu Ser Leu Tyr Pro Gly Asp Thr Ile Lys Ala Ser
            500                 505                 510

Met Leu Asp Ile Thr Arg Asp Pro Leu Arg Glu Ile Ala Leu Glu Thr
        515                 520                 525

Ala Met Thr Gln Arg Lys Leu Arg Asn Phe Phe Gly Pro Glu Phe Val
    530                 535                 540

Lys Met Thr Ile Glu Pro Phe Ile Ser Leu Asp Leu Pro Arg Ser Ile
545                 550                 555                 560

Leu Thr Lys Lys Gly Lys Asn Glu Asp Asn Arg Arg Lys Val Asn Ile
                565                 570                 575

Met Leu Leu Asn Gly Gln Arg Leu Glu Leu Thr Cys Asp Thr Lys Thr
            580                 585                 590

Ile Cys Lys Asp Val Phe Asp Met Val Val Ala His Ile Gly Leu Val
        595                 600                 605

Glu His His Leu Phe Ala Leu Ala Thr Leu Lys Asp Asn Glu Tyr Phe
    610                 615                 620

Phe Val Asp Pro Asp Leu Lys Leu Thr Lys Val Ala Pro Glu Gly Trp
625                 630                 635                 640

Lys Glu Glu Pro Lys Lys Lys Thr Lys Ala Thr Val Asn Phe Thr Leu
                645                 650                 655

Phe Phe Arg Ile Lys Phe Phe Met Asp Asp Val Ser Leu Ile Gln His
            660                 665                 670

Thr Leu Thr Cys His Gln Tyr Tyr Leu Gln Leu Arg Lys Asp Ile Leu
        675                 680                 685

Glu Glu Arg Met His Cys Asp Asp Glu Thr Ser Leu Leu Leu Ala Ser
    690                 695                 700

Leu Ala Leu Gln Ala Glu Tyr Gly Asp Tyr Gln Pro Glu Val His Gly
705                 710                 715                 720

Val Ser Tyr Phe Arg Met Glu His Tyr Leu Pro Ala Arg Val Met Glu
                725                 730                 735

Lys Leu Asp Leu Ser Tyr Ile Lys Glu Glu Leu Pro Lys Leu His Asn
            740                 745                 750

Thr Tyr Val Gly Ala Ser Glu Lys Glu Thr Glu Leu Glu Phe Leu Lys
        755                 760                 765

Val Cys Gln Arg Leu Thr Glu Tyr Gly Val His Phe His Arg Val His
    770                 775                 780

Pro Glu Lys Lys Ser Gln Thr Gly Ile Leu Leu Gly Val Cys Ser Lys
785                 790                 795                 800

Gly Val Leu Val Phe Glu Val His Asn Gly Val Arg Thr Leu Val Leu
                805                 810                 815

Arg Phe Pro Trp Arg Glu Thr Lys Lys Ile Ser Phe Ser Lys Lys Lys
            820                 825                 830

Ile Thr Leu Gln Asn Thr Ser Asp Gly Ile Lys His Gly Phe Gln Thr
        835                 840                 845

Asp Asn Ser Lys Ile Cys Gln Tyr Leu Leu His Leu Cys Ser Tyr Gln
    850                 855                 860

His Lys Phe Gln Leu Gln Met Arg Ala Arg Gln Ser Asn Gln Asp Ala
865                 870                 875                 880

Gln Asp Ile Asp Val Leu His Lys Arg Trp Ser Ile Val Ser Ser Pro
                885                 890                 895

Glu Arg Glu Ile Thr Leu Val Asn Leu Lys Lys Asp Ala Lys Tyr Gly
            900                 905                 910

Leu Gly Phe Gln Ile Ile Gly Gly Glu Lys Met Gly Arg Leu Asp Leu
        915                 920                 925

Gly Ile Phe Ile Ser Ser Val Ala Pro Gly Gly Pro Ala Asp Leu Asp
    930                 935                 940

Gly Cys Leu Lys Pro Gly Asp Arg Leu Ile Ser Val Asn Ser Val Ser
945                 950                 955                 960

Leu Glu Gly Val Ser His His Ala Ala Ile Glu Ile Leu Gln Asn Ala
                965                 970                 975

Pro Glu Asp Val Thr Leu Val Ile Ser Gln Pro Lys Glu Lys Ile Ser
            980                 985                 990

Lys Val Pro Ser Thr Pro Val His  Leu Thr Asn Glu Met  Lys Asn Tyr
        995                 1000                 1005

Met Lys  Lys Ser Ser Tyr Met  Gln Asp Ser Ala Ile  Asp Ser Ser
    1010                 1015                 1020

Ser Lys  Asp His His Trp Ser  Arg Gly Thr Leu Arg  His Ile Ser
    1025                 1030                 1035

Glu Asn  Ser Phe Gly Pro Ser  Gly Gly Leu Arg Glu  Gly Ser Leu
    1040                 1045                 1050

Ser Ser  Gln Asp Ser Arg Thr  Glu Ser Ala Ser Leu  Ser Gln Ser
    1055                 1060                 1065

Gln Val  Asn Gly Phe Phe Ala  Ser His Leu Gly Asp  Gln Thr Trp
    1070                 1075                 1080

Gln Glu Ser Gln His Gly Ser Pro Ser Pro Ser Val Ile Ser Lys
    1085                1090                1095

Ala Thr Glu Lys Glu Thr Phe Thr Asp Ser Asn Gln Ser Lys Thr
    1100                1105                1110

Lys Lys Pro Gly Ile Ser Asp Val Thr Asp Tyr Ser Asp Arg Gly
    1115                1120                1125

Asp Ser Asp Met Asp Glu Ala Thr Tyr Ser Ser Ser Gln Asp His
    1130                1135                1140

Gln Thr Pro Lys Gln Glu Ser Ser Ser Ser Val Asn Thr Ser Asn
    1145                1150                1155

Lys Met Asn Phe Lys Thr Phe Ser Ser Ser Pro Pro Lys Pro Gly
    1160                1165                1170

Asp Ile Phe Glu Val Glu Leu Ala Lys Asn Asp Asn Ser Leu Gly
    1175                1180                1185

Ile Ser Val Thr Gly Gly Val Asn Thr Ser Val Arg His Gly Gly
    1190                1195                1200

Ile Tyr Val Lys Ala Val Ile Pro Gln Gly Ala Ala Glu Ser Asp
    1205                1210                1215

Gly Arg Ile His Lys Gly Asp Arg Val Leu Ala Val Asn Gly Val
    1220                1225                1230

Ser Leu Glu Gly Ala Thr His Lys Gln Ala Val Glu Thr Leu Arg
    1235                1240                1245

Asn Thr Gly Gln Val Val His Leu Leu Leu Glu Lys Gly Gln Ser
    1250                1255                1260

Pro Thr Ser Lys Glu His Val Pro Val Thr Pro Gln Cys Thr Leu
    1265                1270                1275

Ser Asp Gln Asn Ala Gln Gly Gln Gly Pro Glu Lys Val Lys Lys
    1280                1285                1290

Thr Thr Gln Val Lys Asp Tyr Ser Phe Val Thr Glu Glu Asn Thr
    1295                1300                1305

Phe Glu Val Lys Leu Phe Lys Asn Ser Ser Gly Leu Gly Phe Ser
    1310                1315                1320

Phe Ser Arg Glu Asp Asn Leu Ile Pro Glu Gln Ile Asn Ala Ser
    1325                1330                1335

Ile Val Arg Val Lys Lys Leu Phe Pro Gly Gln Pro Ala Ala Glu
    1340                1345                1350

Ser Gly Lys Ile Asp Val Gly Asp Val Ile Leu Lys Val Asn Gly
    1355                1360                1365

Ala Ser Leu Lys Gly Leu Ser Gln Gln Glu Val Ile Ser Ala Leu
    1370                1375                1380

Arg Gly Thr Ala Pro Glu Val Phe Leu Leu Leu Cys Arg Pro Pro
    1385                1390                1395

Pro Gly Val Leu Pro Glu Ile Asp Thr Ala Leu Leu Thr Pro Leu
    1400                1405                1410

Gln Ser Pro Ala Gln Val Leu Pro Asn Ser Ser Lys Asp Ser Ser
    1415                1420                1425

Gln Pro Ser Cys Val Glu Gln Ser Thr Ser Ser Asp Glu Asn Glu
    1430                1435                1440

Met Ser Asp Lys Ser Lys Lys Gln Cys Lys Ser Pro Ser Arg Arg
    1445                1450                1455

Asp Ser Tyr Ser Asp Ser Ser Gly Ser Gly Glu Asp Asp Leu Val
    1460                1465                1470

Thr Ala Pro Ala Asn Ile Ser Asn Ser Thr Trp Ser Ser Ala Leu
    1475                1480                1485

His Gln Thr Leu Ser Asn Met Val Ser Gln Ala Gln Ser His His
    1490                1495                1500

Glu Ala Pro Lys Ser Gln Glu Asp Thr Ile Cys Thr Met Phe Tyr
    1505                1510                1515

Tyr Pro Gln Lys Ile Pro Asn Lys Pro Glu Phe Glu Asp Ser Asn
    1520                1525                1530

Pro Ser Pro Leu Pro Pro Asp Met Ala Pro Gly Gln Ser Tyr Gln
    1535                1540                1545

Pro Gln Ser Glu Ser Ala Ser Ser Ser Ser Met Asp Lys Tyr His
    1550                1555                1560

Ile His His Ile Ser Glu Pro Thr Arg Gln Glu Asn Trp Thr Pro
    1565                1570                1575

Leu Lys Asn Asp Leu Glu Asn His Leu Glu Asp Phe Glu Leu Glu
    1580                1585                1590

Val Glu Leu Leu Ile Thr Leu Ile Lys Ser Glu Lys Gly Ser Leu
    1595                1600                1605

Gly Phe Thr Val Thr Lys Gly Asn Gln Arg Ile Gly Cys Tyr Val
    1610                1615                1620

His Asp Val Ile Gln Asp Pro Ala Lys Ser Asp Gly Arg Leu Lys
    1625                1630                1635

Pro Gly Asp Arg Leu Ile Lys Val Asn Asp Thr Asp Val Thr Asn
    1640                1645                1650

Met Thr His Thr Asp Ala Val Asn Leu Leu Arg Ala Ala Ser Lys
    1655                1660                1665

Thr Val Arg Leu Val Ile Gly Arg Val Leu Glu Leu Pro Arg Ile
    1670                1675                1680

Pro Met Leu Pro His Leu Leu Pro Asp Ile Thr Leu Thr Cys Asn
    1685                1690                1695

Lys Glu Glu Leu Gly Phe Ser Leu Cys Gly Gly His Asp Ser Leu
    1700                1705                1710

Tyr Gln Val Val Tyr Ile Ser Asp Ile Asn Pro Arg Ser Val Ala
    1715                1720                1725

Ala Ile Glu Gly Asn Leu Gln Leu Leu Asp Val Ile His Tyr Val
    1730                1735                1740

Asn Gly Val Ser Thr Gln Gly Met Thr Leu Glu Glu Val Asn Arg
    1745                1750                1755

Ala Leu Asp Met Ser Leu Pro Ser Leu Val Leu Lys Ala Thr Arg
    1760                1765                1770

Asn Asp Leu Pro Val Val Pro Ser Ser Lys Arg Ser Ala Val Ser
    1775                1780                1785

Ala Pro Lys Ser Thr Lys Gly Asn Gly Ser Tyr Ser Val Gly Ser
    1790                1795                1800

Cys Ser Gln Pro Ala Leu Thr Pro Asn Asp Ser Phe Ser Thr Val
    1805                1810                1815

Ala Gly Glu Glu Ile Asn Glu Ile Ser Tyr Pro Lys Gly Lys Cys
    1820                1825                1830

Ser Thr Tyr Gln Ile Lys Gly Ser Pro Asn Leu Thr Leu Pro Lys
    1835                1840                1845

Glu Ser Tyr Ile Gln Glu Asp Asp Ile Tyr Asp Asp Ser Gln Glu
    1850                1855                1860

Ala Glu Val Ile Gln Ser Leu Leu Asp Val Val Asp Glu Glu Ala
    1865                1870                1875

Gln Asn Leu Leu Asn Glu Asn Asn Ala Ala Gly Tyr Ser Cys Gly
    1880                1885                1890

Pro Gly Thr Leu Lys Met Asn Gly Lys Leu Ser Glu Glu Arg Thr
    1895                1900                1905

Glu Asp Thr Asp Cys Asp Gly Ser Pro Leu Pro Glu Tyr Phe Thr
    1910                1915                1920

Glu Ala Thr Lys Met Asn Gly Cys Glu Glu Tyr Cys Glu Glu Lys
    1925                1930                1935

Val Lys Ser Glu Ser Leu Ile Gln Lys Pro Gln Glu Lys Lys Thr
    1940                1945                1950

Asp Asp Asp Glu Ile Thr Trp Gly Asn Asp Glu Leu Pro Ile Glu
    1955                1960                1965

Arg Thr Asn His Glu Asp Ser Asp Lys Asp His Ser Phe Leu Thr
    1970                1975                1980

Asn Asp Glu Leu Ala Val Leu Pro Val Val Lys Val Leu Pro Ser
    1985                1990                1995

Gly Lys Tyr Thr Gly Ala Asn Leu Lys Ser Val Ile Arg Val Leu
    2000                2005                2010

Arg Gly Leu Leu Asp Gln Gly Ile Pro Ser Lys Glu Leu Glu Asn
    2015                2020                2025

Leu Gln Glu Leu Lys Pro Leu Asp Gln Cys Leu Ile Gly Gln Thr
    2030                2035                2040

Lys Glu Asn Arg Arg Lys Asn Arg Tyr Lys Asn Ile Leu Pro Tyr
    2045                2050                2055

Asp Ala Thr Arg Val Pro Leu Gly Asp Glu Gly Gly Tyr Ile Asn
    2060                2065                2070

Ala Ser Phe Ile Lys Ile Pro Val Gly Lys Glu Glu Phe Val Tyr
    2075                2080                2085

Ile Ala Cys Gln Gly Pro Leu Pro Thr Thr Val Gly Asp Phe Trp
    2090                2095                2100

Gln Met Ile Trp Glu Gln Lys Ser Thr Val Ile Ala Met Met Thr
    2105                2110                2115

Gln Glu Val Glu Gly Glu Lys Ile Lys Cys Gln Arg Tyr Trp Pro
    2120                2125                2130

Asn Ile Leu Gly Lys Thr Thr Met Val Ser Asn Arg Leu Arg Leu
    2135                2140                2145

Ala Leu Val Arg Met Gln Gln Leu Lys Gly Phe Val Val Arg Ala
    2150                2155                2160

Met Thr Leu Glu Asp Ile Gln Thr Arg Glu Val Arg His Ile Ser
    2165                2170                2175

His Leu Asn Phe Thr Ala Trp Pro Asp His Asp Thr Pro Ser Gln
    2180                2185                2190

Pro Asp Asp Leu Leu Thr Phe Ile Ser Tyr Met Arg His Ile His
    2195                2200                2205

Arg Ser Gly Pro Ile Ile Thr His Cys Ser Ala Gly Ile Gly Arg
    2210                2215                2220

Ser Gly Thr Leu Ile Cys Ile Asp Val Val Leu Gly Leu Ile Ser
    2225                2230                2235

Gln Asp Leu Asp Phe Asp Ile Ser Asp Leu Val Arg Cys Met Arg
    2240                2245                2250

Leu Gln Arg His Gly Met Val Gln Thr Glu Asp Gln Tyr Ile Phe
    2255                2260                2265

Cys Tyr Gln Val Ile Leu Tyr Val Leu Thr Arg Leu Gln Ala Glu
    2270                2275                2280

Glu Glu Gln Lys Gln Gln Pro Gln Leu Leu Lys
    2285                2290

<210>  7
<211>  2490
<212>  PRT
<213>  Homo sapiens

<400>  7

Met His Val Ser Leu Ala Glu Ala Leu Glu Val Arg Gly Gly Pro Leu
1               5                   10                  15

Gln Glu Glu Glu Ile Trp Ala Val Leu Asn Gln Ser Ala Glu Ser Leu
            20                  25                  30

Gln Glu Leu Phe Arg Lys Val Ser Leu Ala Asp Pro Ala Ala Leu Gly
        35                  40                  45

Phe Ile Ile Ser Pro Trp Ser Leu Leu Leu Leu Pro Ser Gly Ser Val
    50                  55                  60

Ser Phe Thr Asp Glu Asn Ile Ser Asn Gln Asp Leu Arg Ala Phe Thr
65                  70                  75                  80

Ala Pro Glu Val Leu Gln Asn Gln Ser Leu Thr Ser Leu Ser Asp Val
                85                  90                  95

Glu Lys Ile His Ile Tyr Ser Leu Gly Met Thr Leu Tyr Trp Gly Ala
            100                 105                 110

Asp Tyr Glu Val Pro Gln Ser Gln Pro Ile Lys Leu Gly Asp His Leu
        115                 120                 125

Asn Ser Ile Leu Leu Gly Met Cys Glu Asp Val Ile Tyr Ala Arg Val
    130                 135                 140

Ser Val Arg Thr Val Leu Asp Ala Cys Ser Ala His Ile Arg Asn Ser
145                 150                 155                 160

Asn Cys Ala Pro Ser Phe Ser Tyr Val Lys His Leu Val Lys Leu Val
                165                 170                 175

Leu Gly Asn Leu Ser Gly Thr Asp Gln Leu Ser Cys Asn Ser Glu Gln
            180                 185                 190

Lys Pro Asp Arg Ser Gln Ala Ile Arg Asp Arg Leu Arg Gly Lys Gly
        195                 200                 205

Leu Pro Thr Gly Arg Ser Ser Thr Ser Asp Val Leu Asp Ile Gln Lys
    210                 215                 220

Pro Pro Leu Ser His Gln Thr Phe Leu Asn Lys Gly Leu Ser Lys Ser
225                 230                 235                 240

Met Gly Phe Leu Ser Ile Lys Asp Thr Gln Asp Glu Asn Tyr Phe Lys
                245                 250                 255

Asp Ile Leu Ser Asp Asn Ser Gly Arg Glu Asp Ser Glu Asn Thr Phe
            260                 265                 270

Ser Pro Tyr Gln Phe Lys Thr Ser Gly Pro Glu Lys Lys Pro Ile Pro
        275                 280                 285

Gly Ile Asp Val Leu Ser Lys Lys Lys Ile Trp Ala Ser Ser Met Asp
    290                 295                 300

Leu Leu Cys Thr Ala Asp Arg Asp Phe Ser Ser Gly Glu Thr Ala Thr
305                 310                 315                 320

Tyr Arg Arg Cys His Pro Glu Ala Val Thr Val Arg Thr Ser Thr Thr
                325                 330                 335

Pro Arg Lys Lys Glu Ala Arg Tyr Ser Asp Gly Ser Ile Ala Leu Asp
            340                 345                 350

Ile Phe Gly Pro Gln Lys Met Asp Pro Ile Tyr His Thr Arg Glu Leu
        355                 360                 365

Pro Thr Ser Ser Ala Ile Ser Ser Ala Leu Asp Arg Ile Arg Glu Arg
    370                 375                 380

Gln Lys Lys Leu Gln Val Leu Arg Glu Ala Met Asn Val Glu Glu Pro
385                 390                 395                 400

Val Arg Arg Tyr Lys Thr Tyr His Gly Asp Val Phe Ser Thr Ser Ser
                405                 410                 415

Glu Ser Pro Ser Ile Ile Ser Ser Glu Ser Asp Phe Arg Gln Val Arg
            420                 425                 430

Arg Ser Glu Ala Ser Lys Arg Phe Glu Ser Ser Ser Gly Leu Pro Gly
        435                 440                 445

Val Asp Glu Thr Leu Ser Gln Gly Gln Ser Gln Arg Pro Ser Arg Gln
    450                 455                 460

Tyr Glu Thr Pro Phe Glu Gly Asn Leu Ile Asn Gln Glu Ile Met Leu
465                 470                 475                 480

Lys Arg Gln Glu Glu Glu Leu Met Gln Leu Gln Ala Lys Met Ala Leu
                485                 490                 495

Arg Gln Ser Arg Leu Ser Leu Tyr Pro Gly Asp Thr Ile Lys Ala Ser
            500                 505                 510

Met Leu Asp Ile Thr Arg Asp Pro Leu Arg Glu Ile Ala Leu Glu Thr
        515                 520                 525

Ala Met Thr Gln Arg Lys Leu Arg Asn Phe Phe Gly Pro Glu Phe Val
    530                 535                 540

Lys Met Thr Ile Glu Pro Phe Ile Ser Leu Asp Leu Pro Arg Ser Ile
545                 550                 555                 560

Leu Thr Lys Lys Gly Lys Asn Glu Asp Asn Arg Arg Lys Val Asn Ile
                565                 570                 575

Met Leu Leu Asn Gly Gln Arg Leu Glu Leu Thr Cys Asp Thr Lys Thr
            580                 585                 590

Ile Cys Lys Asp Val Phe Asp Met Val Val Ala His Ile Gly Leu Val
        595                 600                 605

Glu His His Leu Phe Ala Leu Ala Thr Leu Lys Asp Asn Glu Tyr Phe
    610                 615                 620

Phe Val Asp Pro Asp Leu Lys Leu Thr Lys Val Ala Pro Glu Gly Trp
625                 630                 635                 640

Lys Glu Glu Pro Lys Lys Lys Thr Lys Ala Thr Val Asn Phe Thr Leu
                645                 650                 655

Phe Phe Arg Ile Lys Phe Phe Met Asp Asp Val Ser Leu Ile Gln His
            660                 665                 670

Thr Leu Thr Cys His Gln Tyr Tyr Leu Gln Leu Arg Lys Asp Ile Leu
        675                 680                 685

Glu Glu Arg Met His Cys Asp Asp Glu Thr Ser Leu Leu Leu Ala Ser
    690                 695                 700

Leu Ala Leu Gln Ala Glu Tyr Gly Asp Tyr Gln Pro Glu Val His Gly
705                 710                 715                 720

Val Ser Tyr Phe Arg Met Glu His Tyr Leu Pro Ala Arg Val Met Glu
                725                 730                 735

Lys Leu Asp Leu Ser Tyr Ile Lys Glu Glu Leu Pro Lys Leu His Asn
            740                 745                 750

Thr Tyr Val Gly Ala Ser Glu Lys Glu Thr Glu Leu Glu Phe Leu Lys
        755                 760                 765

Val Cys Gln Arg Leu Thr Glu Tyr Gly Val His Phe His Arg Val His
    770                 775                 780

Pro Glu Lys Lys Ser Gln Thr Gly Ile Leu Leu Gly Val Cys Ser Lys
785                 790                 795                 800

Gly Val Leu Val Phe Glu Val His Asn Gly Val Arg Thr Leu Val Leu
                805                 810                 815

Arg Phe Pro Trp Arg Glu Thr Lys Lys Ile Ser Phe Ser Lys Lys Lys
            820                 825                 830

Ile Thr Leu Gln Asn Thr Ser Asp Gly Ile Lys His Gly Phe Gln Thr
        835                 840                 845

Asp Asn Ser Lys Ile Cys Gln Tyr Leu Leu His Leu Cys Ser Tyr Gln
    850                 855                 860

His Lys Phe Gln Leu Gln Met Arg Ala Arg Gln Ser Asn Gln Asp Ala
865                 870                 875                 880

Gln Asp Ile Glu Arg Ala Ser Phe Arg Ser Leu Asn Leu Gln Ala Glu
                885                 890                 895

Ser Val Arg Gly Phe Asn Met Gly Arg Ala Ile Ser Thr Gly Ser Leu
            900                 905                 910

Ala Ser Ser Thr Leu Asn Lys Leu Ala Val Arg Pro Leu Ser Val Gln
        915                 920                 925

Ala Glu Ile Leu Lys Arg Leu Ser Cys Ser Glu Leu Ser Leu Tyr Gln
    930                 935                 940

Pro Leu Gln Asn Ser Ser Lys Glu Lys Asn Asp Lys Ala Ser Trp Glu
945                 950                 955                 960

Glu Lys Pro Arg Glu Met Ser Lys Ser Tyr His Asp Leu Ser Gln Ala
                965                 970                 975

Ser Leu Tyr Pro His Arg Lys Asn Val Ile Val Asn Met Glu Pro Pro
            980                 985                 990

Pro Gln Thr Val Ala Glu Leu Val  Gly Lys Pro Ser His  Gln Met Ser
        995                 1000                 1005

Arg Ser  Asp Ala Glu Ser Leu  Ala Gly Val Thr Lys  Leu Asn Asn
    1010                 1015                 1020

Ser Lys  Ser Val Ala Ser Leu  Asn Arg Ser Pro Glu  Arg Arg Lys
    1025                 1030                 1035

His Glu  Ser Asp Ser Ser Ser  Ile Glu Asp Pro Gly  Gln Ala Tyr
    1040                 1045                 1050

Val Leu  Gly Met Thr Met His  Ser Ser Gly Asn Ser  Ser Ser Gln
    1055                 1060                 1065

Val Pro  Leu Lys Glu Asn Asp  Val Leu His Lys Arg  Trp Ser Ile
    1070                 1075                 1080

Val Ser  Ser Pro Glu Arg Glu  Ile Thr Leu Val Asn  Leu Lys Lys
    1085                 1090                 1095

Asp Ala  Lys Tyr Gly Leu Gly  Phe Gln Ile Ile Gly  Gly Glu Lys
    1100                 1105                 1110

Met Gly Arg Leu Asp Leu Gly Ile Phe Ile Ser Ser Val Ala Pro
    1115                1120                1125

Gly Gly Pro Ala Asp Leu Asp Gly Cys Leu Lys Pro Gly Asp Arg
    1130                1135                1140

Leu Ile Ser Val Asn Ser Val Ser Leu Glu Gly Val Ser His His
    1145                1150                1155

Ala Ala Ile Glu Ile Leu Gln Asn Ala Pro Glu Asp Val Thr Leu
    1160                1165                1170

Val Ile Ser Gln Pro Lys Glu Lys Ile Ser Lys Val Pro Ser Thr
    1175                1180                1185

Pro Val His Leu Thr Asn Glu Met Lys Asn Tyr Met Lys Lys Ser
    1190                1195                1200

Ser Tyr Met Gln Asp Ser Ala Ile Asp Ser Ser Ser Lys Asp His
    1205                1210                1215

His Trp Ser Arg Gly Thr Leu Arg His Ile Ser Glu Asn Ser Phe
    1220                1225                1230

Gly Pro Ser Gly Gly Leu Arg Glu Gly Ser Leu Ser Ser Gln Asp
    1235                1240                1245

Ser Arg Thr Glu Ser Ala Ser Leu Ser Gln Ser Gln Val Asn Gly
    1250                1255                1260

Phe Phe Ala Ser His Leu Gly Asp Gln Thr Trp Gln Glu Ser Gln
    1265                1270                1275

His Gly Ser Pro Ser Pro Ser Val Ile Ser Lys Ala Thr Glu Lys
    1280                1285                1290

Glu Thr Phe Thr Asp Ser Asn Gln Ser Lys Thr Lys Lys Pro Gly
    1295                1300                1305

Ile Ser Asp Val Thr Asp Tyr Ser Asp Arg Gly Asp Ser Asp Met
    1310                1315                1320

Asp Glu Ala Thr Tyr Ser Ser Ser Gln Asp His Gln Thr Pro Lys
    1325                1330                1335

Gln Glu Ser Ser Ser Ser Val Asn Thr Ser Asn Lys Met Asn Phe
    1340                1345                1350

Lys Thr Phe Ser Ser Ser Pro Pro Lys Pro Gly Asp Ile Phe Glu
    1355                1360                1365

Val Glu Leu Ala Lys Asn Asp Asn Ser Leu Gly Ile Ser Val Thr
    1370                1375                1380

Val Leu Phe Asp Lys Gly Gly Val Asn Thr Ser Val Arg His Gly
    1385                1390                1395

Gly Ile Tyr Val Lys Ala Val Ile Pro Gln Gly Ala Ala Glu Ser
    1400                1405                1410

Asp Gly Arg Ile His Lys Gly Asp Arg Val Leu Ala Val Asn Gly
    1415                1420                1425

Val Ser Leu Glu Gly Ala Thr His Lys Gln Ala Val Glu Thr Leu
    1430                1435                1440

Arg Asn Thr Gly Gln Val Val His Leu Leu Leu Glu Lys Gly Gln
    1445                1450                1455

Ser Pro Thr Ser Lys Glu His Val Pro Val Thr Pro Gln Cys Thr
    1460                1465                1470

Leu Ser Asp Gln Asn Ala Gln Gly Gln Gly Pro Glu Lys Val Lys
    1475                1480                1485

Lys Thr Thr Gln Val Lys Asp Tyr Ser Phe Val Thr Glu Glu Asn
    1490                1495                1500

Thr Phe Glu Val Lys Leu Phe Lys Asn Ser Ser Gly Leu Gly Phe
    1505                1510                1515

Ser Phe Ser Arg Glu Asp Asn Leu Ile Pro Glu Gln Ile Asn Ala
    1520                1525                1530

Ser Ile Val Arg Val Lys Lys Leu Phe Pro Gly Gln Pro Ala Ala
    1535                1540                1545

Glu Ser Gly Lys Ile Asp Val Gly Asp Val Ile Leu Lys Val Asn
    1550                1555                1560

Gly Ala Ser Leu Lys Gly Leu Ser Gln Gln Glu Val Ile Ser Ala
    1565                1570                1575

Leu Arg Gly Thr Ala Pro Glu Val Phe Leu Leu Leu Cys Arg Pro
    1580                1585                1590

Pro Pro Gly Val Leu Pro Glu Ile Asp Thr Ala Leu Leu Thr Pro
    1595                1600                1605

Leu Gln Ser Pro Ala Gln Val Leu Pro Asn Ser Ser Lys Asp Ser
    1610                1615                1620

Ser Gln Pro Ser Cys Val Glu Gln Ser Thr Ser Ser Asp Glu Asn
    1625                1630                1635

Glu Met Ser Asp Lys Ser Lys Lys Gln Cys Lys Ser Pro Ser Arg
    1640                1645                1650

Arg Asp Ser Tyr Ser Asp Ser Ser Gly Ser Gly Glu Asp Asp Leu
    1655                1660                1665

Val Thr Ala Pro Ala Asn Ile Ser Asn Ser Thr Trp Ser Ser Ala
    1670                1675                1680

Leu His Gln Thr Leu Ser Asn Met Val Ser Gln Ala Gln Ser His
    1685                1690                1695

His Glu Ala Pro Lys Ser Gln Glu Asp Thr Ile Cys Thr Met Phe
    1700                1705                1710

Tyr Tyr Pro Gln Lys Ile Pro Asn Lys Pro Glu Phe Glu Asp Ser
    1715                1720                1725

Asn Pro Ser Pro Leu Pro Pro Asp Met Ala Pro Gly Gln Ser Tyr
    1730                1735                1740

Gln Pro Gln Ser Glu Ser Ala Ser Ser Ser Ser Met Asp Lys Tyr
    1745                1750                1755

His Ile His His Ile Ser Glu Pro Thr Arg Gln Glu Asn Trp Thr
    1760                1765                1770

Pro Leu Lys Asn Asp Leu Glu Asn His Leu Glu Asp Phe Glu Leu
    1775                1780                1785

Glu Val Glu Leu Leu Ile Thr Leu Ile Lys Ser Glu Lys Gly Ser
    1790                1795                1800

Leu Gly Phe Thr Val Thr Lys Gly Asn Gln Arg Ile Gly Cys Tyr
    1805                1810                1815

Val His Asp Val Ile Gln Asp Pro Ala Lys Ser Asp Gly Arg Leu
    1820                1825                1830

Lys Pro Gly Asp Arg Leu Ile Lys Val Asn Asp Thr Asp Val Thr
    1835                1840                1845

Asn Met Thr His Thr Asp Ala Val Asn Leu Leu Arg Ala Ala Ser
    1850                1855                1860

Lys Thr Val Arg Leu Val Ile Gly Arg Val Leu Glu Leu Pro Arg
    1865                1870                1875

Ile Pro Met Leu Pro His Leu Leu Pro Asp Ile Thr Leu Thr Cys
    1880                1885                1890

Asn Lys Glu Glu Leu Gly Phe Ser Leu Cys Gly Gly His Asp Ser
    1895                1900                1905

Leu Tyr Gln Val Val Tyr Ile Ser Asp Ile Asn Pro Arg Ser Val
    1910                1915                1920

Ala Ala Ile Glu Gly Asn Leu Gln Leu Leu Asp Val Ile His Tyr
    1925                1930                1935

Val Asn Gly Val Ser Thr Gln Gly Met Thr Leu Glu Glu Val Asn
    1940                1945                1950

Arg Ala Leu Asp Met Ser Leu Pro Ser Leu Val Leu Lys Ala Thr
    1955                1960                1965

Arg Asn Asp Leu Pro Val Val Pro Ser Ser Lys Arg Ser Ala Val
    1970                1975                1980

Ser Ala Pro Lys Ser Thr Lys Gly Asn Gly Ser Tyr Ser Val Gly
    1985                1990                1995

Ser Cys Ser Gln Pro Ala Leu Thr Pro Asn Asp Ser Phe Ser Thr
    2000                2005                2010

Val Ala Gly Glu Glu Ile Asn Glu Ile Ser Tyr Pro Lys Gly Lys
    2015                2020                2025

Cys Ser Thr Tyr Gln Ile Lys Gly Ser Pro Asn Leu Thr Leu Pro
    2030                2035                2040

Lys Glu Ser Tyr Ile Gln Glu Asp Asp Ile Tyr Asp Asp Ser Gln
    2045                2050                2055

Glu Ala Glu Val Ile Gln Ser Leu Leu Asp Val Val Asp Glu Glu
    2060                2065                2070

Ala Gln Asn Leu Leu Asn Glu Asn Asn Ala Ala Gly Tyr Ser Cys
    2075                2080                2085

Gly Pro Gly Thr Leu Lys Met Asn Gly Lys Leu Ser Glu Glu Arg
    2090                2095                2100

Thr Glu Asp Thr Asp Cys Asp Gly Ser Pro Leu Pro Glu Tyr Phe
    2105                2110                2115

Thr Glu Ala Thr Lys Met Asn Gly Cys Glu Glu Tyr Cys Glu Glu
    2120                2125                2130

Lys Val Lys Ser Glu Ser Leu Ile Gln Lys Pro Gln Glu Lys Lys
    2135                2140                2145

Thr Asp Asp Asp Glu Ile Thr Trp Gly Asn Asp Glu Leu Pro Ile
    2150                2155                2160

Glu Arg Thr Asn His Glu Asp Ser Asp Lys Asp His Ser Phe Leu
    2165                2170                2175

Thr Asn Asp Glu Leu Ala Val Leu Pro Val Val Lys Val Leu Pro
    2180                2185                2190

Ser Gly Lys Tyr Thr Gly Ala Asn Leu Lys Ser Val Ile Arg Val
    2195                2200                2205

Leu Arg Gly Leu Leu Asp Gln Gly Ile Pro Ser Lys Glu Leu Glu
    2210                2215                2220

Asn Leu Gln Glu Leu Lys Pro Leu Asp Gln Cys Leu Ile Gly Gln
    2225                2230                2235

Thr Lys Glu Asn Arg Arg Lys Asn Arg Tyr Lys Asn Ile Leu Pro
    2240                2245                2250

Tyr Asp Ala Thr Arg Val Pro Leu Gly Asp Glu Gly Gly Tyr Ile
    2255                2260                2265

Asn Ala Ser Phe Ile Lys Ile Pro Val Gly Lys Glu Glu Phe Val
    2270                2275                2280

Tyr Ile Ala Cys Gln Gly Pro Leu Pro Thr Thr Val Gly Asp Phe
    2285                2290                2295

Trp Gln Met Ile Trp Glu Gln Lys Ser Thr Val Ile Ala Met Met
    2300                2305                2310

Thr Gln Glu Val Glu Gly Glu Lys Ile Lys Cys Gln Arg Tyr Trp
    2315                2320                2325

Pro Asn Ile Leu Gly Lys Thr Thr Met Val Ser Asn Arg Leu Arg
    2330                2335                2340

Leu Ala Leu Val Arg Met Gln Gln Leu Lys Gly Phe Val Val Arg
    2345                2350                2355

Ala Met Thr Leu Glu Asp Ile Gln Thr Arg Glu Val Arg His Ile
    2360                2365                2370

Ser His Leu Asn Phe Thr Ala Trp Pro Asp His Asp Thr Pro Ser
    2375                2380                2385

Gln Pro Asp Asp Leu Leu Thr Phe Ile Ser Tyr Met Arg His Ile
    2390                2395                2400

His Arg Ser Gly Pro Ile Ile Thr His Cys Ser Ala Gly Ile Gly
    2405                2410                2415

Arg Ser Gly Thr Leu Ile Cys Ile Asp Val Val Leu Gly Leu Ile
    2420                2425                2430

Ser Gln Asp Leu Asp Phe Asp Ile Ser Asp Leu Val Arg Cys Met
    2435                2440                2445

Arg Leu Gln Arg His Gly Met Val Gln Thr Glu Asp Gln Tyr Ile
    2450                2455                2460

Phe Cys Tyr Gln Val Ile Leu Tyr Val Leu Thr Arg Leu Gln Ala
    2465                2470                2475

Glu Glu Glu Gln Lys Gln Gln Pro Gln Leu Leu Lys
    2480                2485                2490

<210>  8
<211>  2466
<212>  PRT
<213>  Homo sapiens

<400>  8

Met His Val Ser Leu Ala Glu Ala Leu Glu Val Arg Gly Gly Pro Leu
1               5                   10                  15

Gln Glu Glu Glu Ile Trp Ala Val Leu Asn Gln Ser Ala Glu Ser Leu
            20                  25                  30

Gln Glu Leu Phe Arg Lys Val Ser Leu Ala Asp Pro Ala Ala Leu Gly
        35                  40                  45

Phe Ile Ile Ser Pro Trp Ser Leu Leu Leu Leu Pro Ser Gly Ser Val
    50                  55                  60

Ser Phe Thr Asp Glu Asn Ile Ser Asn Gln Asp Leu Arg Ala Phe Thr
65                  70                  75                  80

Ala Pro Glu Val Leu Gln Asn Gln Ser Leu Thr Ser Leu Ser Asp Val
                85                  90                  95

Glu Lys Ile His Ile Tyr Ser Leu Gly Met Thr Leu Tyr Trp Gly Ala
            100                 105                 110

Asp Tyr Glu Val Pro Gln Ser Gln Pro Ile Lys Leu Gly Asp His Leu
        115                 120                 125

Asn Ser Ile Leu Leu Gly Met Cys Glu Asp Val Ile Tyr Ala Arg Val

130                 135                 140

Ser Val Arg Thr Val Leu Asp Ala Cys Ser Ala His Ile Arg Asn Ser
145                 150                 155                 160

Asn Cys Ala Pro Ser Phe Ser Tyr Val Lys His Leu Val Lys Leu Val
                165                 170                 175

Leu Gly Asn Leu Ser Gly Thr Asp Gln Leu Ser Cys Asn Ser Glu Gln
            180                 185                 190

Lys Pro Asp Arg Ser Gln Ala Ile Arg Asp Arg Leu Arg Gly Lys Gly
        195                 200                 205

Leu Pro Thr Gly Arg Ser Ser Thr Ser Asp Val Leu Asp Ile Gln Lys
    210                 215                 220

Pro Pro Leu Ser His Gln Thr Phe Leu Asn Lys Gly Leu Ser Lys Ser
225                 230                 235                 240

Met Gly Phe Leu Ser Ile Lys Asp Thr Gln Asp Glu Asn Tyr Phe Lys
                245                 250                 255

Asp Ile Leu Ser Asp Asn Ser Gly Arg Glu Asp Ser Glu Asn Thr Phe
            260                 265                 270

Ser Pro Tyr Gln Phe Lys Thr Ser Gly Pro Glu Lys Lys Pro Ile Pro
        275                 280                 285

Gly Ile Asp Val Leu Ser Lys Lys Lys Ile Trp Ala Ser Ser Met Asp
    290                 295                 300

Leu Leu Cys Thr Ala Asp Arg Asp Phe Ser Ser Gly Glu Thr Ala Thr
305                 310                 315                 320

Tyr Arg Arg Cys His Pro Glu Ala Val Thr Val Arg Thr Ser Thr Thr
                325                 330                 335

Pro Arg Lys Lys Glu Ala Arg Tyr Ser Asp Gly Ser Ile Ala Leu Asp
            340                 345                 350

Ile Phe Gly Pro Gln Lys Met Asp Pro Ile Tyr His Thr Arg Glu Leu
        355                 360                 365

Pro Thr Ser Ser Ala Ile Ser Ser Ala Leu Asp Arg Ile Arg Glu Arg
    370                 375                 380

Gln Lys Lys Leu Gln Val Leu Arg Glu Ala Met Asn Val Glu Glu Pro
385                 390                 395                 400

Val Arg Arg Tyr Lys Thr Tyr His Gly Asp Val Phe Ser Thr Ser Ser

                405                 410                 415

Glu Ser Pro Ser Ile Ile Ser Ser Glu Ser Asp Phe Arg Gln Val Arg
            420                 425                 430

Arg Ser Glu Ala Ser Lys Arg Phe Glu Ser Ser Ser Gly Leu Pro Gly
        435                 440                 445

Val Asp Glu Thr Leu Ser Gln Gly Gln Ser Gln Arg Pro Ser Arg Gln
    450                 455                 460

Tyr Glu Thr Pro Phe Glu Gly Asn Leu Ile Asn Gln Glu Ile Met Leu
465                 470                 475                 480

Lys Arg Gln Glu Glu Glu Leu Met Gln Leu Gln Ala Lys Met Ala Leu
                485                 490                 495

Arg Gln Ser Arg Leu Ser Leu Tyr Pro Gly Asp Thr Ile Lys Ala Ser
            500                 505                 510

Met Leu Asp Ile Thr Arg Asp Pro Leu Arg Glu Ile Ala Leu Glu Thr
        515                 520                 525

Ala Met Thr Gln Arg Lys Leu Arg Asn Phe Phe Gly Pro Glu Phe Val
    530                 535                 540

Lys Met Thr Ile Glu Pro Phe Ile Ser Leu Asp Leu Pro Arg Ser Ile
545                 550                 555                 560

Leu Thr Lys Lys Gly Lys Asn Glu Asp Asn Arg Arg Lys Val Asn Ile
                565                 570                 575

Met Leu Leu Asn Gly Gln Arg Leu Glu Leu Thr Cys Asp Thr Lys Thr
            580                 585                 590

Ile Cys Lys Asp Val Phe Asp Met Val Val Ala His Ile Gly Leu Val
        595                 600                 605

Glu His His Leu Phe Ala Leu Ala Thr Leu Lys Asp Asn Glu Tyr Phe
    610                 615                 620

Phe Val Asp Pro Asp Leu Lys Leu Thr Lys Val Ala Pro Glu Gly Trp
625                 630                 635                 640

Lys Glu Glu Pro Lys Lys Lys Thr Lys Ala Thr Val Asn Phe Thr Leu
                645                 650                 655

Phe Phe Arg Ile Lys Phe Phe Met Asp Asp Val Ser Leu Ile Gln His
            660                 665                 670

Thr Leu Thr Cys His Gln Tyr Tyr Leu Gln Leu Arg Lys Asp Ile Leu

675                 680                 685

Glu Glu Arg Met His Cys Asp Asp Glu Thr Ser Leu Leu Leu Ala Ser
    690                 695                 700

Leu Ala Leu Gln Ala Glu Tyr Gly Asp Tyr Gln Pro Glu Val His Gly
705                 710                 715                 720

Val Ser Tyr Phe Arg Met Glu His Tyr Leu Pro Ala Arg Val Met Glu
                725                 730                 735

Lys Leu Asp Leu Ser Tyr Ile Lys Glu Glu Leu Pro Lys Leu His Asn
            740                 745                 750

Thr Tyr Val Gly Ala Ser Glu Lys Glu Thr Glu Leu Glu Phe Leu Lys
        755                 760                 765

Val Cys Gln Arg Leu Thr Glu Tyr Gly Val His Phe His Arg Val His
    770                 775                 780

Pro Glu Lys Lys Ser Gln Thr Gly Ile Leu Leu Gly Val Cys Ser Lys
785                 790                 795                 800

Gly Val Leu Val Phe Glu Val His Asn Gly Val Arg Thr Leu Val Leu
                805                 810                 815

Arg Phe Pro Trp Arg Glu Thr Lys Lys Ile Ser Phe Ser Lys Lys Lys
            820                 825                 830

Ile Thr Leu Gln Asn Thr Ser Asp Gly Ile Lys His Gly Phe Gln Thr
        835                 840                 845

Asp Asn Ser Lys Ile Cys Gln Tyr Leu Leu His Leu Cys Ser Tyr Gln
    850                 855                 860

His Lys Phe Gln Leu Gln Met Arg Ala Arg Gln Ser Asn Gln Asp Ala
865                 870                 875                 880

Gln Asp Ile Glu Arg Ala Ser Phe Arg Ser Leu Asn Leu Gln Ala Glu
                885                 890                 895

Ser Val Arg Gly Phe Asn Met Gly Arg Ala Ile Ser Thr Gly Ser Leu
            900                 905                 910

Ala Ser Ser Thr Leu Asn Lys Leu Ala Val Arg Pro Leu Ser Val Gln
        915                 920                 925

Ala Glu Ile Leu Lys Arg Leu Ser Cys Ser Glu Leu Ser Leu Tyr Gln
    930                 935                 940

Pro Leu Gln Asn Ser Ser Lys Glu Lys Asn Asp Lys Ala Ser Trp Glu

945                 950                 955                 960

Glu Lys Pro Arg Glu Met Ser Lys Ser Tyr His Asp Leu Ser Gln Ala
                965                 970                 975

Ser Leu Tyr Pro His Arg Lys Asn Val Ile Val Asn Met Glu Pro Pro
            980                 985                 990

Pro Gln Thr Val Ala Glu Leu Val  Gly Lys Pro Ser His  Gln Met Ser
        995                 1000                 1005

Arg Ser  Asp Ala Glu Ser Leu  Ala Gly Val Thr Lys  Leu Asn Asn
    1010                 1015                 1020

Ser Lys  Ser Val Ala Ser Leu  Asn Arg Ser Pro Glu  Arg Arg Lys
    1025                 1030                 1035

His Glu  Ser Asp Ser Ser Ser  Ile Glu Asp Pro Gly  Gln Ala Tyr
    1040                 1045                 1050

Val Leu  Asp Val Leu His Lys  Arg Trp Ser Ile Val  Ser Ser Pro
    1055                 1060                 1065

Glu Arg  Glu Ile Thr Leu Val  Asn Leu Lys Lys Asp  Ala Lys Tyr
    1070                 1075                 1080

Gly Leu  Gly Phe Gln Ile Ile  Gly Gly Glu Lys Met  Gly Arg Leu
    1085                 1090                 1095

Asp Leu  Gly Ile Phe Ile Ser  Ser Val Ala Pro Gly  Gly Pro Ala
    1100                 1105                 1110

Asp Leu  Asp Gly Cys Leu Lys  Pro Gly Asp Arg Leu  Ile Ser Val
    1115                 1120                 1125

Asn Ser  Val Ser Leu Glu Gly  Val Ser His His Ala  Ala Ile Glu
    1130                 1135                 1140

Ile Leu  Gln Asn Ala Pro Glu  Asp Val Thr Leu Val  Ile Ser Gln
    1145                 1150                 1155

Pro Lys  Glu Lys Ile Ser Lys  Val Pro Ser Thr Pro  Val His Leu
    1160                 1165                 1170

Thr Asn  Glu Met Lys Asn Tyr  Met Lys Lys Ser Ser  Tyr Met Gln
    1175                 1180                 1185

Asp Ser  Ala Ile Asp Ser Ser  Ser Lys Asp His His  Trp Ser Arg
    1190                 1195                 1200

Gly Thr  Leu Arg His Ile Ser  Glu Asn Ser Phe Gly  Pro Ser Gly

1205                    1210                    1215

Gly Leu  Arg Glu Gly Ser Leu  Ser Ser Gln Asp Ser  Arg Thr Glu
    1220                    1225                    1230

Ser Ala  Ser Leu Ser Gln Ser  Gln Val Asn Gly Phe  Phe Ala Ser
    1235                    1240                    1245

His Leu  Gly Asp Gln Thr Trp  Gln Glu Ser Gln His  Gly Ser Pro
    1250                    1255                    1260

Ser Pro  Ser Val Ile Ser Lys  Ala Thr Glu Lys Glu  Thr Phe Thr
    1265                    1270                    1275

Asp Ser  Asn Gln Ser Lys Thr  Lys Lys Pro Gly Ile  Ser Asp Val
    1280                    1285                    1290

Thr Asp  Tyr Ser Asp Arg Gly  Asp Ser Asp Met Asp  Glu Ala Thr
    1295                    1300                    1305

Tyr Ser  Ser Ser Gln Asp His  Gln Thr Pro Lys Gln  Glu Ser Ser
    1310                    1315                    1320

Ser Ser  Val Asn Thr Ser Asn  Lys Met Asn Phe Lys  Thr Phe Ser
    1325                    1330                    1335

Ser Ser  Pro Pro Lys Pro Gly  Asp Ile Phe Glu Val  Glu Leu Ala
    1340                    1345                    1350

Lys Asn  Asp Asn Ser Leu Gly  Ile Ser Val Thr Gly  Gly Val Asn
    1355                    1360                    1365

Thr Ser  Val Arg His Gly Gly  Ile Tyr Val Lys Ala  Val Ile Pro
    1370                    1375                    1380

Gln Gly  Ala Ala Glu Ser Asp  Gly Arg Ile His Lys  Gly Asp Arg
    1385                    1390                    1395

Val Leu  Ala Val Asn Gly Val  Ser Leu Glu Gly Ala  Thr His Lys
    1400                    1405                    1410

Gln Ala  Val Glu Thr Leu Arg  Asn Thr Gly Gln Val  Val His Leu
    1415                    1420                    1425

Leu Leu  Glu Lys Gly Gln Ser  Pro Thr Ser Lys Glu  His Val Pro
    1430                    1435                    1440

Val Thr  Pro Gln Cys Thr Leu  Ser Asp Gln Asn Ala  Gln Gly Gln
    1445                    1450                    1455

Gly Pro  Glu Lys Val Lys Lys  Thr Thr Gln Val Lys  Asp Tyr Ser

1460                    1465                    1470

Phe Val  Thr Glu Glu Asn Thr  Phe Glu Val Lys Leu  Phe Lys Asn
    1475                    1480                    1485

Ser Ser  Gly Leu Gly Phe Ser  Phe Ser Arg Glu Asp  Asn Leu Ile
    1490                    1495                    1500

Pro Glu  Gln Ile Asn Ala Ser  Ile Val Arg Val Lys  Lys Leu Phe
    1505                    1510                    1515

Pro Gly  Gln Pro Ala Ala Glu  Ser Gly Lys Ile Asp  Val Gly Asp
    1520                    1525                    1530

Val Ile  Leu Lys Val Asn Gly  Ala Ser Leu Lys Gly  Leu Ser Gln
    1535                    1540                    1545

Gln Glu  Val Ile Ser Ala Leu  Arg Gly Thr Ala Pro  Glu Val Phe
    1550                    1555                    1560

Leu Leu  Leu Cys Arg Pro Pro  Pro Gly Val Leu Pro  Glu Ile Asp
    1565                    1570                    1575

Thr Ala  Leu Leu Thr Pro Leu  Gln Ser Pro Ala Gln  Val Leu Pro
    1580                    1585                    1590

Asn Ser  Ser Lys Asp Ser Ser  Gln Pro Ser Cys Val  Glu Gln Ser
    1595                    1600                    1605

Thr Ser  Ser Asp Glu Asn Glu  Met Ser Asp Lys Ser  Lys Lys Gln
    1610                    1615                    1620

Cys Lys  Ser Pro Ser Arg Arg  Asp Ser Tyr Ser Asp  Ser Ser Gly
    1625                    1630                    1635

Ser Gly  Glu Asp Asp Leu Val  Thr Ala Pro Ala Asn  Ile Ser Asn
    1640                    1645                    1650

Ser Thr  Trp Ser Ser Ala Leu  His Gln Thr Leu Ser  Asn Met Val
    1655                    1660                    1665

Ser Gln  Ala Gln Ser His His  Glu Ala Pro Lys Ser  Gln Glu Asp
    1670                    1675                    1680

Thr Ile  Cys Thr Met Phe Tyr  Tyr Pro Gln Lys Ile  Pro Asn Lys
    1685                    1690                    1695

Pro Glu  Phe Glu Asp Ser Asn  Pro Ser Pro Leu Pro  Pro Asp Met
    1700                    1705                    1710

Ala Pro  Gly Gln Ser Tyr Gln  Pro Gln Ser Glu Ser  Ala Ser Ser

1715                    1720                    1725

Ser Ser Met Asp Lys Tyr His Ile His His Ile Ser Glu Pro Thr
    1730                1735                1740

Arg Gln Glu Asn Trp Thr Pro Leu Lys Asn Asp Leu Glu Asn His
    1745                1750                1755

Leu Glu Asp Phe Glu Leu Glu Val Glu Leu Leu Ile Thr Leu Ile
    1760                1765                1770

Lys Ser Glu Lys Gly Ser Leu Gly Phe Thr Val Thr Lys Gly Asn
    1775                1780                1785

Gln Arg Ile Gly Cys Tyr Val His Asp Val Ile Gln Asp Pro Ala
    1790                1795                1800

Lys Ser Asp Gly Arg Leu Lys Pro Gly Asp Arg Leu Ile Lys Val
    1805                1810                1815

Asn Asp Thr Asp Val Thr Asn Met Thr His Thr Asp Ala Val Asn
    1820                1825                1830

Leu Leu Arg Ala Ala Ser Lys Thr Val Arg Leu Val Ile Gly Arg
    1835                1840                1845

Val Leu Glu Leu Pro Arg Ile Pro Met Leu Pro His Leu Leu Pro
    1850                1855                1860

Asp Ile Thr Leu Thr Cys Asn Lys Glu Glu Leu Gly Phe Ser Leu
    1865                1870                1875

Cys Gly Gly His Asp Ser Leu Tyr Gln Val Val Tyr Ile Ser Asp
    1880                1885                1890

Ile Asn Pro Arg Ser Val Ala Ala Ile Glu Gly Asn Leu Gln Leu
    1895                1900                1905

Leu Asp Val Ile His Tyr Val Asn Gly Val Ser Thr Gln Gly Met
    1910                1915                1920

Thr Leu Glu Glu Val Asn Arg Ala Leu Asp Met Ser Leu Pro Ser
    1925                1930                1935

Leu Val Leu Lys Ala Thr Arg Asn Asp Leu Pro Val Val Pro Ser
    1940                1945                1950

Ser Lys Arg Ser Ala Val Ser Ala Pro Lys Ser Thr Lys Gly Asn
    1955                1960                1965

Gly Ser Tyr Ser Val Gly Ser Cys Ser Gln Pro Ala Leu Thr Pro

1970                    1975                    1980

Asn Asp Ser Phe Ser Thr Val Ala Gly Glu Glu Ile Asn Glu Ile
    1985                    1990                    1995

Ser Tyr Pro Lys Gly Lys Cys Ser Thr Tyr Gln Ile Lys Gly Ser
    2000                    2005                    2010

Pro Asn Leu Thr Leu Pro Lys Glu Ser Tyr Ile Gln Glu Asp Asp
    2015                    2020                    2025

Ile Tyr Asp Asp Ser Gln Glu Ala Glu Val Ile Gln Ser Leu Leu
    2030                    2035                    2040

Asp Val Val Asp Glu Glu Ala Gln Asn Leu Leu Asn Glu Asn Asn
    2045                    2050                    2055

Ala Ala Gly Tyr Ser Cys Gly Pro Gly Thr Leu Lys Met Asn Gly
    2060                    2065                    2070

Lys Leu Ser Glu Glu Arg Thr Glu Asp Thr Asp Cys Asp Gly Ser
    2075                    2080                    2085

Pro Leu Pro Glu Tyr Phe Thr Glu Ala Thr Lys Met Asn Gly Cys
    2090                    2095                    2100

Glu Glu Tyr Cys Glu Glu Lys Val Lys Ser Glu Ser Leu Ile Gln
    2105                    2110                    2115

Lys Pro Gln Glu Lys Lys Thr Asp Asp Asp Glu Ile Thr Trp Gly
    2120                    2125                    2130

Asn Asp Glu Leu Pro Ile Glu Arg Thr Asn His Glu Asp Ser Asp
    2135                    2140                    2145

Lys Asp His Ser Phe Leu Thr Asn Asp Glu Leu Ala Val Leu Pro
    2150                    2155                    2160

Val Val Lys Val Leu Pro Ser Gly Lys Tyr Thr Gly Ala Asn Leu
    2165                    2170                    2175

Lys Ser Val Ile Arg Val Leu Arg Gly Leu Leu Asp Gln Gly Ile
    2180                    2185                    2190

Pro Ser Lys Glu Leu Glu Asn Leu Gln Glu Leu Lys Pro Leu Asp
    2195                    2200                    2205

Gln Cys Leu Ile Gly Gln Thr Lys Glu Asn Arg Arg Lys Asn Arg
    2210                    2215                    2220

Tyr Lys Asn Ile Leu Pro Tyr Asp Ala Thr Arg Val Pro Leu Gly

2225                    2230                    2235

Asp Glu  Gly Gly Tyr Ile Asn  Ala Ser Phe Ile Lys  Ile Pro Val
    2240                    2245                    2250

Gly Lys  Glu Glu Phe Val Tyr  Ile Ala Cys Gln Gly  Pro Leu Pro
    2255                    2260                    2265

Thr Thr  Val Gly Asp Phe Trp  Gln Met Ile Trp Glu  Gln Lys Ser
    2270                    2275                    2280

Thr Val  Ile Ala Met Met Thr  Gln Glu Val Glu Gly  Glu Lys Ile
    2285                    2290                    2295

Lys Cys  Gln Arg Tyr Trp Pro  Asn Ile Leu Gly Lys  Thr Thr Met
    2300                    2305                    2310

Val Ser  Asn Arg Leu Arg Leu  Ala Leu Val Arg Met  Gln Gln Leu
    2315                    2320                    2325

Lys Gly  Phe Val Val Arg Ala  Met Thr Leu Glu Asp  Ile Gln Thr
    2330                    2335                    2340

Arg Glu  Val Arg His Ile Ser  His Leu Asn Phe Thr  Ala Trp Pro
    2345                    2350                    2355

Asp His  Asp Thr Pro Ser Gln  Pro Asp Asp Leu Leu  Thr Phe Ile
    2360                    2365                    2370

Ser Tyr  Met Arg His Ile His  Arg Ser Gly Pro Ile  Ile Thr His
    2375                    2380                    2385

Cys Ser  Ala Gly Ile Gly Arg  Ser Gly Thr Leu Ile  Cys Ile Asp
    2390                    2395                    2400

Val Val  Leu Gly Leu Ile Ser  Gln Asp Leu Asp Phe  Asp Ile Ser
    2405                    2410                    2415

Asp Leu  Val Arg Cys Met Arg  Leu Gln Arg His Gly  Met Val Gln
    2420                    2425                    2430

Thr Glu  Asp Gln Tyr Ile Phe  Cys Tyr Gln Val Ile  Leu Tyr Val
    2435                    2440                    2445

Leu Thr  Arg Leu Gln Ala Glu  Glu Glu Gln Lys Gln  Gln Pro Gln
    2450                    2455                    2460

Leu Leu  Lys
    2465

<210>  9

<211>  21
<212>  DNA
<213>  artificial sequence

<220>
<223>  PTP gamma upper primer

<400>  9
gcaactcgat ggcttcgaca a                                                21


<210>  10
<211>  24
<212>  DNA
<213>  artificial sequence

<220>
<223>  PTP gamma lower primer

<400>  10
tctttcagaa ggatggcgac tgtt                                             24


<210>  11
<211>  21
<212>  DNA
<213>  artificial sequence

<220>
<223>  LAR-Neu upper primer

<400>  11
tggactcccc atcatccaag a                                                21


<210>  12
<211>  25
<212>  DNA
<213>  artificial sequence

<220>
<223>  LAR-Neu lower primer

<400>  12
ccgctgatag tggtttcata gtcct                                            25


<210>  13
<211>  19
<212>  DNA
<213>  artificial sequence

<220>
<223>  LAR upper primer

<400>  13
tagccgaggc ccaggaaac                                                   19


<210>  14
<211>  21
<212>  DNA
<213>  artificial sequence

<220>
<223>  LAR lower primer

<400>  14

ccccttggtgg tataggcagc a                                                21

<210>  15
<211>  19
<212>  DNA
<213>  artificial sequence

<220>
<223>  PTPL1 upper primer

<400>  15
caaaggtgat cgcgtccta                                                    19

<210>  16
<211>  22
<212>  DNA
<213>  artificial sequence

<220>
<223>  PTPL1 lower primer

<400>  16
cgggacatgt tctttagatg tt                                                22

<210>  17
<211>  18
<212>  DNA
<213>  artificial sequence

<220>
<223>  HPRTupper primer

<400>  17
ctgacctgct ggattaca                                                     18

<210>  18
<211>  18
<212>  DNA
<213>  artificial sequence

<220>
<223>  HPRTlower primer

<400>  18
gcgaccttga ccatcttt                                                     18